# EUROPEAN PATENT APPLICATION

(11) **EP 1 842 549 A2**
(43) Date of publication of application: **10.10.2007**
(21) Application number: 07013019.0
(22) Date of filing: 28.12.2000
(51) Int. Cl.: A61K 38/00, A01N 43/04

(54) **Optimized minigenes and peptides encoded thereby**

(30) Priority: 28.12.1999 US 173390 P
(62) Divisional of application: 00989585.5
(71) Applicant: Pharmexa Inc., San Diego, CA 92121 (US)
(72) Inventor: Sette, Alessandro, La Jolla, CA 92037 (US); Chesnut, Robert, Cardiff-by-the-Sea, CA 92007 (US); Livingston, Brian D., San Diego, CA 92129 (US); Baker, Denise Marie, Poway, CA 92064 (US); Newman, Mark J., Carlsbad, CA 92009 (US)
(74) Representative: Spencer, Matthew Peter

(57) **Abstract**

This present invention relates to the field of biology. In particular, it relates to polyepitopic vaccines and methods of designing such vaccines to provide increased immunogenicity. In certain embodiments, the polyepitopic vaccines is encoded by a minigene that provides optimized immunogenicity of the construct.

## Description

### CROSS-REFERENCES TO RELATED APPLICATIONS

The present application claims priority to U.S. Provisional Application No. 60/173,390 filed December 28, 1999, which is hereby incorporated by reference.

### STATEMENT AS TO RIGHTS TO INVENTIONS MADE UNDER FEDERALLY SPONSORED RESEARCH AND DEVELOPMENT

This invention was made with Federal Funding assistance. Accordingly, the U.S. government may have certain rights to the invention.

### BACKGROUND OF THE INVENTION

This present invention relates to the field of biology. In particular, it relates to polyepitopic vaccines and methods of designing such vaccines to provide increased immunogenicity. In certain embodiments, the polyepitopic vaccines is encoded by a minigene that provides optimized immunogenicity of the construct.

The technology relevant to multi-epitope ("minigene") vaccines is developing. Several independent studies have established that induction of simultaneous immune responses against multiple epitopes can be achieved. For example, responses against a large number of T cell specificities can be induced and detected. In natural situations, Doolan et al [Immunity, Vol. 7(1):97-112 (1997)] simultaneously detected recall T cell responses, against as many as 17 different *P. falciparum* epitopes using PBMC from a single donor. Similarly, Bertoni and colleagues [J Clin Invest, Vol. 100(3):503-13 (1997)] detected simultaneous responses against 12 different HBV-derived epitopes in a single donor. In terms of immunization with multieptope DNA minigene vaccines, several examples have been reported where multiple T cell responses were induced. For example, minigene vaccines composed of approximately ten MHC class I epitopes in which all epitopes were immunogenic and/or antigenic have been reported. Specifically, minigene vaccines composed of 9 EBV [Thomson et al., Proc Natl Acad Sci USA, Vol. 92(13):5845-9 (1995)], 7 HIV [Woodberry et al., J Virol, Vol. 73(7):5320-5 (1999)], 10 murine [Thomson et al., J Immunol, Vol. 160(4):1717-23 (1998)] and 10 tumor- derived [Mateo et al., J Immunol, Vol. 163(7):4058-63 (1999)] epitopes have been shown to be active. It has also been shown that a multi-epitope DNA plasmid encoding nine different HLA-A2.1- and A11-restricted epitopes derived from the HBV and HIV induced CTL against all epitopes [Ishioka et al., J Immunol, Vol. 162(7):3915-25 (1999)].

Thus, minigene vaccines containing multiple MHC Class I (*i.e.*, CTL) epitopes can be designed, and presentation and recognition can be obtained for all epitopes. However, the immunogenicity of multi-epitope constructs appears to be strongly influenced by a number of variables, a number of which have heretofore been unknown. For example, the immunogenicity (or antigenicity) of the same epitope expressed in the context of different vaccine constructs can vary over several orders of magnitude. Thus, there exists a need to identify strategies to optimize multiepitope vaccine constructs. Such optimization is important in terms of induction of potent immune responses and ultimately, for clinical efficacy. Accordingly, the present invention provides strategies to optimize antigenicity and immunogenicity of polyepitopic vaccines encompassing a large number of epitopes, and optimized polyepitopic vaccines, particularly minigene vaccines, generated in accordance with these stratesgies.

The following paragraphs provide a brief review of some of the main variables potentially influencing minigene immunogenicity, epitope processing, and presentation on antigen presenting cells (APCs) in association with Class I and Class II MHC molecules.

### Immunodominance

Of the many thousand possible peptides that are encoded by a complex foreign pathogen, only a small fraction ends up in a peptide form capable of binding to MHC Class I antigens and thus of being recognized by T cells. This phenomenon, of obvious potential impact on the development of a multi-epitope vaccine, is known as immunodominance [Yewdell et al., Annu Rev Immunol, 17:51-88 (1999)]. Several major variables contribute to immunodominance. Herein, we describe variables affecting the generation of the appropriate peptides, both in qualitative and quantitative terms, as a result of intracellular processing.

### Junctional Epitopes

A junctional epitope is defined as an epitope created due to the juxtaposition of two other epitopes. The new epitope is composed of a C-terminal section derived from a first epitope, and an N-terminal section derived from a second epitope. Creation of junctional epitopes is a potential problem in the design of multiepitope minigene vaccines, for both Class I and Class II restricted epitopes for the following reasons. Firstly, when developing a minigene composed of, or containing, human epitopes, which are typically tested for immunogenicity in HLA transgenic laboratory animals, the creation of murine epitopes could create undesired immunodominance effects. Secondly, the creation of new, unintended epitopes for human HLA Class I or Class II molecules could elicit in vaccine recipients, new T cell specificities that are not expressed by infected cells or tumors that are the targets-induced T cell responses. These responses are by definition irrelevant and ineffective and could even be counterproductive, by creating undesired immunodominance effects.

The existence of junctional epitopes has been documented in a variety of different experimental situations. Gefter and collaborators first demonstrated the effect in a system in which two different Class II restricted epitopes were juxtaposed and colinearly synthesized [Perkins et al., J Immunol, Vol. 146(7):2137-44 (1991)]. The effect was so marked that the immune system recognition of the epitopes could be completely "silenced" by these new junctional epitopes [Wang et al., Cell Immunol, Vol. 143(2):284-97 (1992)]. Helper T cells directed against junctional epitopes were also observed in humans as a result of immunization with a synthetic lipopeptide, which was composed of an HLA-A2-restricted HBV-derived immunodominant CTL epitope, and a universal Tetanus Toxoid-derived HTL epitope [Livingston et al, J Immunol, Vol. 159(3):1383-92 (1997)]. Thus, the creation of junctional epitopes are a major consideration in the design of polyepitopic constructs.

The present invention provides methods of addressing this problem and avoiding or minimizing the occurrence of junctional epitopes.

### Flanking regions

Class I restricted epitopes are generated by a complex process [Yewdell et al., Annu Rev Immunol, 17:51-88 (1999)]. Limited proteolysis involving endoproteases and potential trimming by exoproteases is followed by translocation across the endoplasmic reticulum (ER) membrane by transporter associated with antigen processing (TAP) molecules. The major cytosolic protease complex involved in generation of antigenic peptides, and their precursors, is the proteosome [Niedermann et al., Immunity, Vol. 2(3):289-99 (1995)], although ER trimming of CTL precursors has also been demonstrated [Paz et al., Immunity Vol. 11(2):241-51 (1999)]. It has long been debated whether or not the residues immediately flanking the C and N terminus of the epitope, have an influence on the efficiency of epitope generation.

The yield and availability of processed epitope has been implicated as a major variable in determining immunogenicity and could thus clearly have a major impact on overall minigene potency in that the magnitude of immune response can be directly proportional to the amount of epitope bound by MHC and displayed for T cell recognition. Several studies have provided evidence that this is indeed the case. For example, induction of virus-specific CTL that is essentially proportional to epitope density [Wherry et al., J Immunol, Vol. 163(7):3735-45 (1999)] has been observed. Further, recombinant minigenes, which encode a preprocessed optimal epitope, have been used to induce higher levels of epitope expression than naturally observed with full-length protein [Anton et al., J Immunol, Vol. 158(6):2535-42 (1997)]. In general, minigene priming has been shown to be more effective than priming with the whole antigen [Restifo et al., J Immunol, Vol. 154(9):4414-22 (1995); Ishioka et al., J Immunol, Vol. 162(7):3915-25 (1999)], even though some exceptions have been noted [Iwasaki et al., Vaccine, Vol. 17(15-16):2081-8 (1999)].

Early studies concluded that residues within the epitope [Hahn et al., J Exp Med, Vol. 176(5):1335-41 (1992)] primarily regulate immunogenicity. Similar conclusions were reached by other studies, mostly based on grafting an epitope in an unrelated gene, or in the same gene, but in a different location [Chimini et al., J Exp Med, Vol. 169(1):297-302 (1989); Hahn et al., J Exp Med, Vol. 174(3):733-6 (1991)]. Other experiments however [Del Val et al., Cell, Vol. 66(6):1145-53 (1991); Hahn et al., J Exp Med, Vol. 176(5):1335-41 (1992)], suggested that residues localized directly adjacent to the CTL epitope can directly influence recognition [Couillin et al., J Exp Med, Vol. 180(3):1129-34 (1994); Bergmann et al., J Virol. Vol. 68(8):5306-10 (1994)]. In the context of minigene vaccines, the controversy has been renewed. Shastri and coworkers [Shastri et al., J Immunol, Vol. 155(9):4339-46 (1995)] have found that T cell responses were not significantly affected by varying the N-terminal flanking residue but were inhibited by the addition of a single C-terminal flanking residue. The most dramatic inhibition was observed with isoleucine, leucine, cysteine, and proline as the C-terminal flanking residues. In contrast, Gileadi [Gileadi et al., Eur J Immunol, Vol. 29(7):2213-22 (1999)] reported profound effects as a function of the residues located at the N terminus of mouse influenza virus epitopes. Bergmann and coworkers found that aromatic, basic and alanine residues supported efficient epitope recognition, while G and P residues were strongly inhibitory [Bergmann et al., J Immunol, Vol. 157(8):3242-9 (1996)]. In contrast, Lippolis [Lippolis et al., J Virol, Vol. 69(5):3134-46 (1995)] concluded that substituting flanking residues did not effect recognition. However, only rather conservative substitutions, unlikely to affect proteosome specificity, were tested.

It appears that the specificity of these effects, and in general of natural epitopes, roughly correlates with proteosome specificity. For example, proteosome specificity is partly trypsin-like [ Niedermann et al., Immunity, Vol. 2(3):289-99 (1995)], with cleavage following basic amino acids. Nevertheless, efficient cleavage of the carboxyl side of hydrophobic and acidic residues is also possible. Consistent with these specificities are the studies of Sherman and collaborators, which found that an R to H mutation at the position following the C-terminus of a p53 epitope affects proteosome-mediated processing of the protein [Theobald et al., J Exp Med, Vol. 188(6):1017-28 (1998)]. Several other studies [Hanke et al., J Gen Virol, Vol. 79 (Pt 1):83-90 (1998); Thomson et al., Proc Natl Acad Sci USA, Vol. 92(13):5845-9 (1995)] indicated that minigenes can be constructed utilizing minimal epitopes, and that these flanking sequences appear not be required, although the potential for further optimization by the use of flanking regions was also acknowledged.

In sum, for HLA Class I epitopes, the effects of flanking regions on processing and presentation of CTL epitopes is as yet undefined. A systematic analysis of the effect of modulation of flanking regions has not been performed for minigene vaccines. Thus, analysis utilizing minigene vaccines encoding epitopes restricted by human Class I in general is needed. The present invention provides such an analysis and accordingly, provides polyepitopic vaccine constructs optimized for immunogenicity and antigenicity, and methods of designing such constructs.

HLA Class II peptide complexes are also generated as a result of a complex series of events that is distinct from HLA Class I processing. The processing pathway involves association with Invariant chain (Ii), its transport to specialized compartments, the degradation of Ii to CLIP, and HLA-DM catalyzed removal of CLIP (see [Blum et al., Crit Rev Immunol, Vol. 17(5-6):411-7 (1997); Amdt et al., Immunol Res, Vol. 16(3):261-72 (1997)] for review,) Moreover, there is a potential crucial role of various cathepsins in general, and cathepsin S and L in particular, in Ii degradation [Nakagawa et al., Immunity, Vol. 10(2):207-17 (1999)]. In terms of generation of functional epitopes however, the process appears to be somewhat less selective [Chapman H.A., Curr Opin Immunol, Vol. 10(1):93-102 (1998)], and peptides of many sizes can bind to MHC Class II [Hunt et al., Science, Vol. 256(5065):1817-20 (1992)]. Most or all of the possible peptides appear to be generated [Moudgil et al., J Immunol, Vol. 159(6):2574-9 (1997); and Thomson et al., J Virol, Vol. 72(3):2246-52 (1998)]. Thus, as compared to the issue of flanking regions, the creation of junctional epitopes can be a more serious concern in particular embodiments.

### SUMMARY OF THE INVENTION

This invention provides disclosure of parameters useful to optimize the efficacy of polyepitopic vaccines. Also disclosed are polyepitopic constructs and nucleicic acids encoding such constructs (minigenes).

In one aspect, the invention provides a method for designing a polyepitopic construct that comprises multiple HLA epitopes and is presented to an HLA Class I processing pathway, the method comprising steps of: (i) sorting the multiple HLA epitopes to minimize the number of junctional epitopes; (ii) introducing a flanking amino acid residue selected from the group consisting of K, R, N, Q, G, A, S, C, and T at a C+1 position of an HLA epitope comprised by the polyepitopic construct; (iii) introducing one or more amino acid spacer residues between two epitopes comprised by the polyepitopic construct, wherein the spacer prevents the occurrence of a CTL or HTL junctional epitope; and, (iv) selecting one or more polyepitopic constructs that have a minimal number of junctional epitopes, a minimal number of amino acid spacer residues, and a maximum number of K, R, N, G,A, S., C, or T at a C+1 position relative to each HLA epitope. In some embodimes, the spacer residues are independently selected from residues that are not known HLA Class II primary anchor residues. In particular embodiments, ntroducing the spacer residues prevents the occurrence of an HTL epitope. Such a spacer often comprises at least 5 amino acid residues independently selected from the group consisting of G, P, and N. In some embodiments the spacer is GPGPG.

In some embodiments, introducing the spacer residues prevents the occurrence of a CTL epitope and further, wherein the spacer is 1, 2, 3, 4, 5, 6, 7, or 8 amino acid residues independently selected from the group consisting of A and G. Often, the flanking residue is introduced at the C+1 position of a CTL epitope and is selected from the group consisting of K, R, N, G, and A.

In some embodiments, the flanking residue is adjacent to the spacer sequence. The methods of the invention can also include substituting an N-terminal residue of an HLA epitope that is adjacent to a C-terminus of an HLA epitope comprised by the polyepitopic construct with a residue selected from the group consisting of K, R, N, G, and A.

The methods of the invention can also further comprise a step of predicting a structure of the polyepitopic construct, and further, selecting one or more constructs that have a maximal structure, *i.e.*, that are processed by an HLA processing pathway to produce all of the epitopes comprised by the construct.

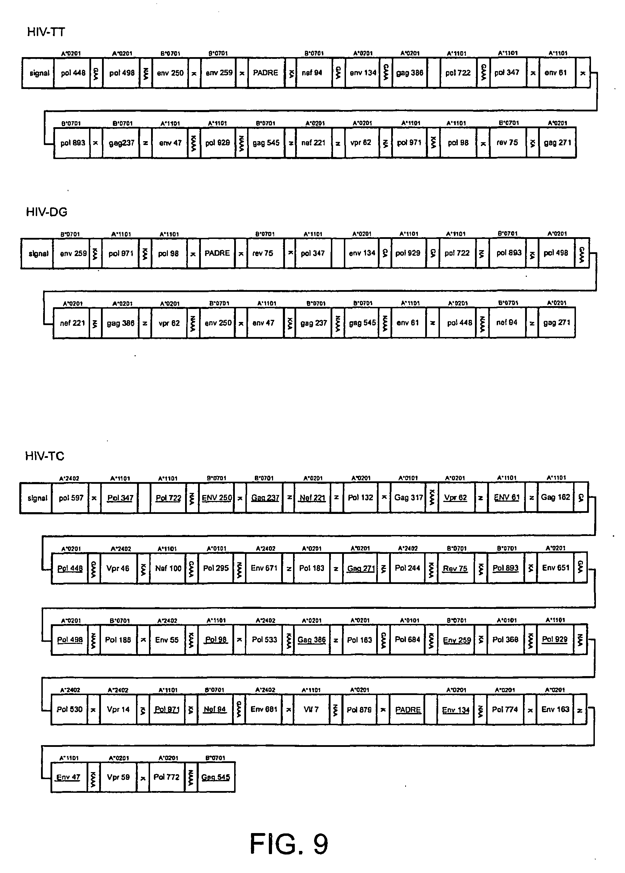

In another apsect, the invention provides a polyepitopic construct prepared using a method in accordance with any of claims 1-9. Often, the epitopes comprised by the polyepitopic construct are encoded by a minigene. In preferred embodiments, the polyepitopic construct encoded by the minigene is HIV-TT as set out in Figure 9, HIV-DG as set out in Figure 9, or HIV-TC as set out in Figure 9.

### DEFINITIONS

The invention can be better understood with reference to the following definitions:

Throughout this disclosure, "binding data" results are often expressed in terms of "IC₅₀'s." IC₅₀ is the concentration of peptide in a binding assay at which 50% inhibition of binding of a reference peptide is observed. Given the conditions in which the assays are run (i. e., limiting HLA proteins and labeled peptide concentrations), these values approximate K_{D} values. Assays for determining binding are described in detail, e.g., in PCT publications WO 94/20127 and WO 94/03205. It should be noted that IC₅₀ values can change, often dramatically, if the assay conditions are varied, and depending on the particular reagents used (e.g., HLA preparation, *etc.).* For example, excessive concentrations of HLA molecules will increase the apparent measured IC₅₀ of a given ligand. Alternatively, binding is expressed relative to a reference peptide. Although as a particular assay becomes more, or less, sensitive, the IC₅₀'s of the peptides tested may change somewhat, the binding relative to the reference peptide will not significantly change. For example, in an assay run under conditions such that the IC₅₀ of the reference peptide increases 10-fold, the IC₅₀ values of the test peptides will also shift approximately 10-fold. Therefore, to avoid ambiguities, the assessment of whether a peptide is a good, intermediate, weak, or negative binder is generally based on its IC₅₀, relative to the IC₅₀ of a standard peptide. Binding may also be determined using other assay systems including those using: live cells (e.g., Ceppellini et al., Nature 339:392, 1989; Christnick et al., Nature 352:67, 1991; Busch et al., Int. Immunol. 2:443, 19990; Hill et al., J. Immunol. 147:189, 1991; del Guercio et al., J. Immunol. 154:685, 1995), cell free systems using detergent lysates (e.g., Cerundolo et al., J. Immunol. 21:2069, 1991), immobilized purified MHC *(e.g.,* Hill et al., J. Immunol. 152, 2890, 1994; Marshall et al., J. Immunol. 152:4946, 1994), ELISA systems (e.g., Reay et al., EMBO J. 11:2829, 1992), surface plasmon resonance (*e.g.,* Khilko et al., J. Biol. Chem. 268:15425, 1993); high flux soluble phase assays (Hammer et al., J. Exp. Med. 180:2353, 1994), and measurement of class I MHC stabilization or assembly (e.g., Ljunggren et al., Nature 346:476, 1990; Schumacher et al., Cell 62:563, 1990; Townsend et al., Cell 62:285, 1990; Parker et al., J. Immunol. 149:1896, 1992).

The designation of a residue position in an epitope as the "carboxyl terminus" or the "carboxyl terminal position" refers to the residue position at the end of the epitope that is nearest to the carboxyl terminus of a peptide, which is designated using conventional nomenclature as defined below. "C + 1" refers to the residue or position immediately following the C-terminal residue of the epitope, *i*.*e*., refers to the residue flanking the C-terminus of the epitope. The "carboxyl terminal position" of the epitope occurring at the carboxyl end of the polyepitopic construct may or may not actually correspond to the carboxyl terminal end of polypeptide. In preferred embodiments, the epitopes employed in the optimized polyepitopic constructs are motif-bearing epitopes and the carboxyl terminus of the epitope is defined with respect to primary anchor residues corresponding to a particular motif.

The designation of a residue position in an epitope as "amino terminus" or "amino-terminal position" refers to the residue position at the end of the epitope which is nearest to the amino terminus of a peptide, which is designated using conventional nomenclature as defined below. "N-1" refers to the residue or position immediately adjacent to the epitope at the amino terminal end (position number 1) of an eptiope. The "amino terminal position" of the epitope occurring at the amino terminal end of the polyepitopic construct may or may not actually corresponds to the amino terminal end of the polypeptide. In preferred embodiments, the epitopes employed in the optimized polyepitopic constructs are motif-bearing epitopes and the amino terminus of the epitope is defined with respect to primary anchor residues corresponding to a particular motif.

A "computer" or "computer system" generally includes: a processor; at least one information storage/retrieval apparatus such as, for example, a hard drive, a disk drive or a tape drive; at least one input apparatus such as, for example, a keyboard, a mouse, a touch screen, or a microphone; and display structure. Additionally, the computer may include a communication channel in communication with a network. Such a computer may include more or,less than what is listed above.

A "construct" as used herein generally denotes a composition that does not occur in nature. A construct can be produced by synthetic technologies, e.g., recombinant DNA preparation and expression or chemical synthetic techniques for nucleic or amino acids. A construct can also be produced by the addition or affiliation of one material with another such that the result is not found in nature in that form. A "polyepitopic construct" comprises multipe epitopes.

"Cross-reactive binding" indicates that a peptide is bound by more than one HLA molecule; a synonym is degenerate binding.

A "cryptic epitope" elicits a response by immunization with an isolated peptide, but the response is not cross-reactive *in vitro* when intact whole protein that comprises the epitope is used as an antigen.

A "dominant epitope" is an epitope that induces an immune response upon immunization with a whole native antigen (see, e.g., Sercarz, et al., Annu. Rev. Immunol. 11:729-766, 1993). Such a response is cross-reactive *in vitro* with an isolated peptide epitope.

With regard to a particular amino acid sequence, an "epitope" is a set of amino acid residues which is involved in recognition by a particular immunoglobulin, or in the context of T cells, those residues necessary for recognition by T cell receptor proteins and/or Major Histocompatibility Complex (MHC) receptors. In an immune system setting, *in vitro* or *in vitro,* an epitope is the collective features of a molecule, such as primary, secondary and tertiary peptide structure, and charge, that together form a site recognized by an immunoglobulin, T cell receptor or HLA molecule. Throughout this disclosure epitope and peptide are often used interchangeably. It is to be appreciated, however, that isolated or purified protein or peptide molecules larger than and comprising an epitope of the invention are still within the bounds of the invention.

A "flanking residue" is a residue that is positioned next to an eptiope. A flanking residue can be introduced or inserted at at a position adjacent to the N-terminus or the C-terminus of an epitope.

An "immunogenic peptide" or "peptide epitope" is a peptide that comprises an allele-specific motif or supermotif such that the peptide will bind an HLA molecule and induce a CTL and/or HTL response. Thus, immunogenic peptides of the invention are capable of binding to an appropriate HLA molecule and thereafter inducing a cytotoxic T cell response, or a helper T cell response, to the antigen from which the immunogenic peptide is derived. ,

"Heteroclitic analogs" are defined herein as a peptide with increased potency for a specific T cell, as measured by increased responses to a given dose, or by a requirement of lesser amounts to achieve the same response. Advantages of heteroclitic analogs include that the antigens can be more potent, or more economical (since a lower amount is required to achieve the same effect). In addition, modified epitopes might overcoming antigen-specific T cell unresponsiveness (T cell tolerance).

"Human Leukocyte Antigen" or "HLA" is a human class I or class II Major Histocompatibility Complex (MHC) protein (*see, e.g.,* Stites, et al., IMMUNOLOGY, 8TH ED., Lange Publishing, Los Altos, CA (1994).

An "HLA supertype or HLA family," as used herein, describes sets of HLA molecules grouped based on shared peptide-binding specificities. HLA class I molecules that share somewhat similar binding affinity for peptides bearing certain amino acid motifs are grouped into such HLA supertypes. The terms HLA superfamily, HLA supertype family, HLA family, and HLA xx-like molecules (where xx denotes a particular HLA type), are synonyms.

As used herein, "high affinity" with respect to HLA class I molecules is defined as binding with an IC₅₀, or K_{D} value, of 50 nM or less; "intermediate affinity" is binding with an IC₅₀ or K_{D} value of between about 50 and about 500 nM. "High affinity" with respect to binding to HLA class II molecules is defined as binding with an IC₅₀ or K_{D} value of 100 nM or less; "intermediate affinity" is binding with an IC₅₀ or K_{D} value of between about 100 and about 1000 nM.

An "IC₅₀" is the concentration of peptide in a binding assay at which 50% inhibition of binding of a reference peptide is observed. Given the conditions in which the assays are run (i.e., limiting HLA proteins and labeled peptide concentrations), these values approximate K_{D} values.

The terms "identical" or percent "identity," in the context of two or more peptide sequences, refer to two or more sequences or subsequences that are the same or have a specified percentage of amino acid residues that are the same, when compared and aligned for maximum correspondence over a comparison window, as measured using a sequence comparison algorithm or by manual alignment and visual inspection.

"Introducing" an amino acid residue at a particular position in a polyepitopic construct, e.g., adjacent, at the C-terminal side, to the C-terminus of the epitope, encompasses configuring multiple epitopes such that a desired residue is at a particular position, *e.g.,* adjacent to the epitope, or such that a deleterious residue is not adjacent to the C-terminus of the epitope. The term also includes inserting an amino acid residue, preferably a preferred or intermediate amino acid residue, at a particular position. An amino acid residue can also be introduced into a sequence by substituting one amino acid residue for another. Preferably, such a substitution is made in accordance with analoging principles set forth, e.g., in co-pending U.S.S.N. 09/260,714 filed 3/1199 and application PCT/US00/19774.

The phrases "isolated" or "biologically pure" refer to material that is substantially or essentially free from components which normally accompany the material as it is found in its native state. Thus, isolated peptides in accordance with the invention preferably do not contain materials normally associated with the peptides in their *in situ* environment.

"Link" or "join" refers to any method known in the art for functionally connecting peptides, including, without limitation, recombinant fusion, covalent bonding, disulfide bonding, ionic bonding, hydrogen bonding, and electrostatic bonding.

"Major Histocompatibility Complex" or ''MHC'' is a cluster of genes that plays a role in control of the cellular interactions responsible for physiologic immune responses. In humans, the MHC complex is also known as the HLA complex. For a detailed description of the MHC and HLA complexes, see, Paul, FUNDAMENTAL IMMUNOLOGY, 3^{RD} ED., Raven Press, New York, 1993.

As used herein, "middle of the peptide" is a position in a peptide that is neither an amino or a carboxyl terminus.

A "minimal number of junctional epitopes" as used herein refers to a number of junctional epioptes that is lower than what would be created using a random selection criteria.

The term "motif" refers to the pattern of residues in a peptide of defined length, usually a peptide of from about 8 to about 13 amino acids for a class I HLA motif and from about 6 to about 25 amino acids for a class II HLA motif, which is recognized by a particular HLA molecule. Peptide motifs are typically different for each protein encoded by each human HLA allele and differ in the pattern of the primary and secondary anchor residues.

A "negative binding residue" or "deleterious residue" is an amino acid which, if present at certain positions (typically not primary anchor positions) in a peptide epitope, results in decreased binding affinity of the peptide for the peptide's corresponding HLA molecule.

"Optimizing" a polyepitopic construct refers to increasing the immunogenicity or antigenicity of a construct by sorting epitopes to minimize the occurrence of junctional epitopes, inserting flanking residues that flank the C-terminus or N-terminus of an epitope, and inserting spacer residue to further prevent the occurrence of junctional epitopes or to provide a flanking residue. An increase in immunogenicity or antigenicity of an optimized polyepitopic construct is measured relative to a polyepitopic construct that has not been constructed based on the optimization parameters and is using assays known to those of skill in the art, e.g., assessment of immunogenicity in transgenic animals, ELISPOT, inteferon-gamma releast assays, tetramer staining, chromium release assays, and presentation on dendritic cells.

The term "peptide" is used interchangeably with "oligopeptide" in the present specification to designate a series of residues, typically L-amino acids, connected one to the other, typically by peptide bonds between the α-amino and carboxyl groups of adjacent amino acids. The preferred CTL-inducing peptides of the invention are 13 residues or less in length and usually consist of between about 8 and about 11 residues, preferably 9 or 10 residues. The preferred HTL-inducing oligopeptides are less than about 50 residues in length and usually consist of between about 6 and about 30 residues, more usually between about 12 and 25, and often between about 15 and 20 residues.

A "PanDR binding peptide or PADRE^{™} peptide" is a member of a family of molecules that binds more that one HLA class II DR molecule. The pattern that defines the PADRE^{™} family of molecules can be thought of as an HLA Class II supermotif. PADRE binds to most HLA-DR molecules and stimulates *in vitro* and *in vitro* human helper T lymphocyte (HTL) responses.

"Pharmaceutically acceptable" refers to a generally non-toxic, inert, and/or physiologically compatible composition.

"Presented to an HLA Class I processing pathway" means that the polyepitopic constructs are introduced into a cell such that they are largely processed by and HLA Class I processing pathway. Typically, polyepitopic constructs are introduced into the cells using expression vectors that encode the polyepitopic constructs. HLA Class II epitopes that are encoded by such a minigene are also presented on Class II molecules, although the mechanism of entry of the epitopes into the Class II processing pathway is not defined.

A "primary anchor residue" or a "primary MHC anchor" is an amino acid at a specific position along a peptide sequence that is understood to provide a contact point between the immunogenic peptide and the HLA molecule. One to three, usually two, primary anchor residues within a peptide of defined length generally defines a "motif" for an immunogenic peptide. These residues are understood to fit in close contact with peptide binding grooves of an HLA molecule, with their side chains buried in specific pockets of the binding grooves themselves. In one embodiment, for example, the primary anchor residues of an HLA class I epitope are located at position 2 (from the amino terminal position) and at the carboxyl terminal position of a 9-residue peptide epitope in accordance with the invention. The primary anchor positions for each motif and supermotif are decribed, for example, in Tables I and III of PCT/US00/27766, or PCT/US00/19774. Furthermore, analog peptides can be created by altering the presence or absence of particular residues in these primary anchor positions. Such analogs are used to modulate the binding affinity of a peptide comprising a particular motif or supermotif.

"Promiscuous recognition" occurs where a distinct peptide is recognized by the same T cell clone in the context of various HLA molecules. Promiscuous recognition or binding is synonymous with cross-reactive binding.

A "protective immune response" or "therapeutic immune response" refers to a CTL and/or an HTL response to an antigen derived from an infectious agent or a tumor antigen, which in some way prevents or at least partially arrests disease symptoms, side effects or progression. The immune response may also include an antibody response that has been facilitated by the stimulation of helper T cells.

The term "residue" refers to an amino acid or amino acid mimetic incorporated into an peptide pr protein by an amide bond or amide bond mimetic.

A "secondary anchor residue" is an amino acid at a position other than a primary anchor position in a peptide that may influence peptide binding. A secondary anchor residue occurs at a significantly higher frequency amongst bound peptides than would be expected by random distribution of amino acids at one position. The secondary anchor residues are said to occur at "secondary anchor positions." A secondary anchor residue can be identified as a residue which is present at a higher frequency among high or intermediate affinity binding peptides, or a residue otherwise associated with high or intermediate affinity binding. For example, analog peptides can be created by altering the presence or absence of particular residues in these secondary anchor positions. Such analogs are used to finely modulate the binding affinity of a peptide comprising a particular motif or supermotif. The terminology "fixed peptide" is sometimes used to refer to an analog peptide.

"Sorting epitopes" refers to determining or designing an order of the epitopes in a polyepitopic construct.

A "spacer" refers to a sequence that is inserted between two epitopes in a polyepitopic construct to prevent the occurrence of junctional epitopes. For HLA Class II epitopes, the spacer is five amino acids or longer in length, and the amino acid residues in the spacer sequence, such as G, P, or N typically are not known to be primary anchor residues, of an HLA Class II motif *(see, e.g.,* PCT/US00/19774).

A "subdominant epitope" is an epitope which evokes little or no response upon immunization with whole antigens which comprise the epitope, but for which a response can be obtained by immunization with an isolated peptide, and this response (unlike the case of cryptic epitopes) is detected when whole protein is used to recall the response *in vitro* or *in vitro.*

A "supermotif" is a peptide binding specificity shared by HLA molecules encoded by two or more HLA alleles. Preferably, a supermotif-bearing peptide is recognized with high or intermediate affinity (as defined herein) by two or more HLA antigens.

"Synthetic peptide" refers to a peptide that is not naturally occurring, but is man-made using such methods as chemical synthesis or recombinant DNA technology.

A "TCR contact residue" or "T cell receptor contact residue" is an amino acid residue in an epitope that is understood to be bound by a T cell receptor; these are defined herein as not being any primary MHC anchor. T cell receptor contact residues are defined as the position/positions in the peptide where all analogs tested induce negative IFNγ production relative to that induced with wildtype peptide

The nomenclature used to describe peptide compounds follows the conventional practice wherein the amino group is presented to the left (the N-terminus) and the carboxyl group to the right (the C-terminus) of each amino acid residue. When amino acid residue positions are referred to in a peptide epitope they are numbered in an amino to carboxyl direction with position one being the position closest to the amino terminal end of the epitope, or the peptide or protein of which it may be a part. In the formulae representing selected specific embodiments of the present invention, the amino- and carboxyl-terminal groups, although not specifically shown, are in the form they would assume at physiologic pH values, unless otherwise specified. In the amino acid structure formulae, each residue is generally represented by standard three letter or single letter designations. The L-form of an amino acid residue is represented by a capital single letter or a capital first letter of a three-letter symbol, and the D-form for those amino acids having D-forms is represented by a lower case single letter or a lower case three letter symbol. Glycine has no asymmetric carbon atom and is simply referred to as "Gly" or G. Symbols for the amino acids are shown below.

| **Single Letter Symbol** | **Three Letter Symbol** | **Amino Acids** |
|---|---|---|
| A | Ala | Alanine |
| C | Cys | Cysteine |
| D | Asp | Aspartic Acid |
| E | Glu | Glutamic Acid |
| F | Phe | Phenylalanine |
| G | Gly | Glycine |
| H | His | Histidine |
| I | He | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |

Amino acid "chemical characteristics" are defined as: Aromatic (F,W, Y); Aliphatic-hydrophobic (L, I, V, M); Small polar (S, T, C); Large polar (Q, N); Acidic (D, E); Basic (R, H, K); Proline; Alanine; and Glycine.

### Acronyms used herein are as follows:

- APC:: Antigen presenting cell
- CD3:: Pan T cell marker
- CD4:: Helper T lymphocyte marker
- CD8:: Cytotoxic T lymphocyte marker
- CEA:: Carcinoembryonic antigen
- CTL:: Cytotoxic T lymphocytes
- DC:: Dendritic cells. DC functioned as potent antigen presenting cells by stimulating cytokine release from CTL lines that were specific for a model peptide derived from hepatitis B virus (HBV). *In vitro* experiments using DC pulsed *ex vivo* with an HBV peptide epitope have stimulated CTL immune responses *in vitro* following delivery to naive mice.
- DMSO:: Dimethylsulfoxide
- ELISA:: Enzyme-linked immunosorbant assay
- E:T:: Effector:target ratio
- FCS:: Fetal calf serum
- G-CSF:: Granulocyte colony-stimulating factor
- GM-CSF:: Granulocyte-macrophage (monocyte)-colony stimulating factor
- HBV:: Hepatitis B virus
- HER2/Neu:: c-erbB-2
- HLA:: Human leukocyte antigen

- HLA-DR:: Human leukocyte antigen class II
- HPLC:: High Performance Liquid Chromatography
- HTC:: Helper T cells
- HTL:: Helper T Lymphocyte
- ID:: Identity
- IFNγ:: Interferon gamma
- IL-4:: Interleukin-4 cytokine
- IV:: Intravenous
- LU_{30%}:: Cytotoxic activity required to achieve 30% lysis at a 100:1 (E:T) ratio
- MAb:: Monoclonal antibody
- MAGE:: Melanoma antigen
- MLR:: Mixed lymphocyte reaction
- MNC:: Mononuclear cells
- PB:: Peripheral blood
- PBMC:: Peripheral blood mononuclear cell
- SC:: Subcutaneous
- S.E.M.:: Standard error of the mean
- QD:: Once a day dosing
- TAA:: Tumor associated antigen
- TCR:: T cell receptor
- TNF:: Tumor necrosis factor
- WBC:: White blood cells

This application may be relevant to U.S.S.N. 09/189,702 filed 11/10/98, which is a CIP of U.S.S.N 08/205,713 filed 3/4/94, which is a CIP of 08/159,184 filed 11/29/93 and now abandoned, which is a CIP of 08/073,205 filed 6/4/93 and now abandoned, which is a CIP of 08/027,146 filed 3/5/93 and now abandoned. The present application is also related to U.S.S.N. 09/226,775, which is a CIP of U.S.S.N. 08/815,396, which claims the benefit ofU.S.S.N. 60/013,113, now abandoned. Furthermore, the present application is related to U.S.S.N. 09/017,735, which is a CIP of abandoned U.S.S.N. 08/589,108; U.S.S.N. 08/753,622, U.S.S.N. 08/822,382, abandoned U.S.S.N. 60/013,980, U.S.S.N. 08/454,033, U.S.S.N. 09/116,424, and U.S.S.N. 08/349,177. The present application is also related to U.S.S.N. 09/017,524, U.S.S.N. 08/821,739, abandoned U.S.S.N. 60/013,833, U.S.S.N. 08/758,409, U.S.S.N. 08/589,107, U.S.S.N. 08/451,913, U.S.S.N. 08/186,266, U.S.S.N. 09/116,061, and U.S.S.N. 08/347,610, which is a CIP of U.S.S.N. 08/159,339, which is a CIP of abandoned U.S.S.N. 08/103,396, which is a CIP of abandoned U.S.S.N. 08/027,746, which is a CIP of abandoned U.S.S.N. 07/926,666. The present application may also be relevant to U.S.S.N. 09/017,743, U.S.S.N. 08/753,615; U.S.S.N. 08/590,298, U.S.S.N. 09/115,400, and U.S.S.N. 08/452,843, which is a CIP of U.S.S.N. 08/344,824, which is a CIP of abandoned U.S.S.N. 08/278,634. The present application may also be related to provisional U.S.S.N. 60/087,192 and U.S.S.N. 09/009,953, which is a CIP of abandoned U.S.S.N. 60/036,713 and abandoned U.S.S.N. 60/037,432. In addition, the present application may be relevant to U.S.S.N. 09/098,584, and U.S.S.N. 09/239,043. The present application may also be relevant to co-pending U.S.S.N. 09/583,200 filed 5/30/00, U.S.S.N. 09/260,714 filed 3/1/99, and U.S. Provisional Application "Heteroclitic Analogs And Related Methods", Attorney Docket Number 018623-015810US filed 10/6/00 . All of the above applications are incorporated herein by reference.

### BRIEF DESCRIPTION OF THE DRAWINGS

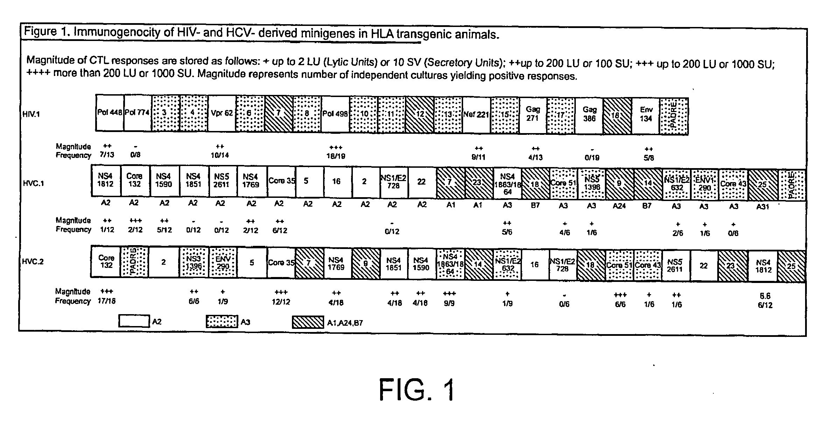

Figure 1 illustrates data on three different multi-epitope constructs, incorporating 20 to 25 different CTL epitopes each.

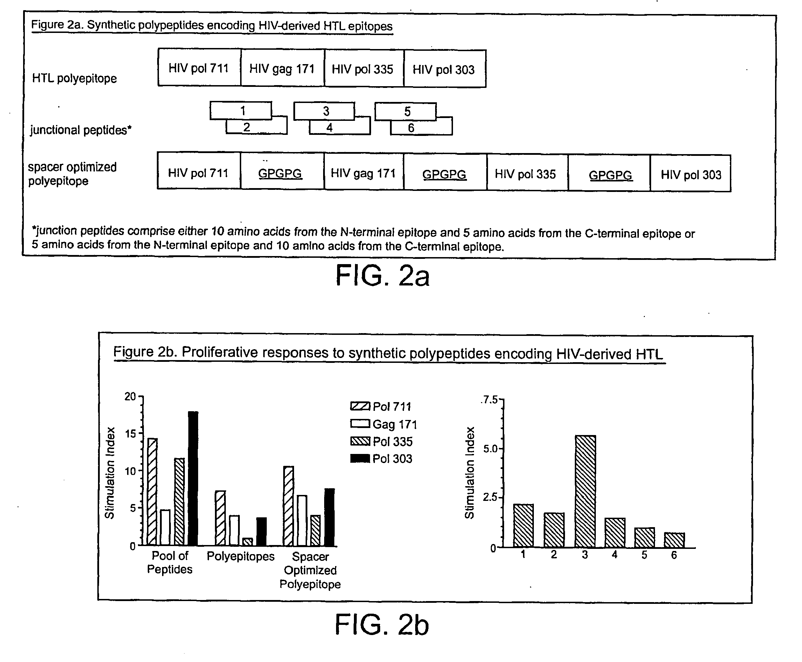

Figure 2 illustrates two different synthetic polypeptides (Fig. 2a) where the first construct incorporates four different epitopes linearly cosynthetized, and the second construct incorporates a GPGPG spacer. Fig. 2b illustrates the capacity of 2 nanomoles of these different constructs to prime for proliferative responses to the various epitopes in IA^{b} positive mice, compared to the responses induced by equimolar amounts of a pool of the same peptides (3 micrograms of each peptide).

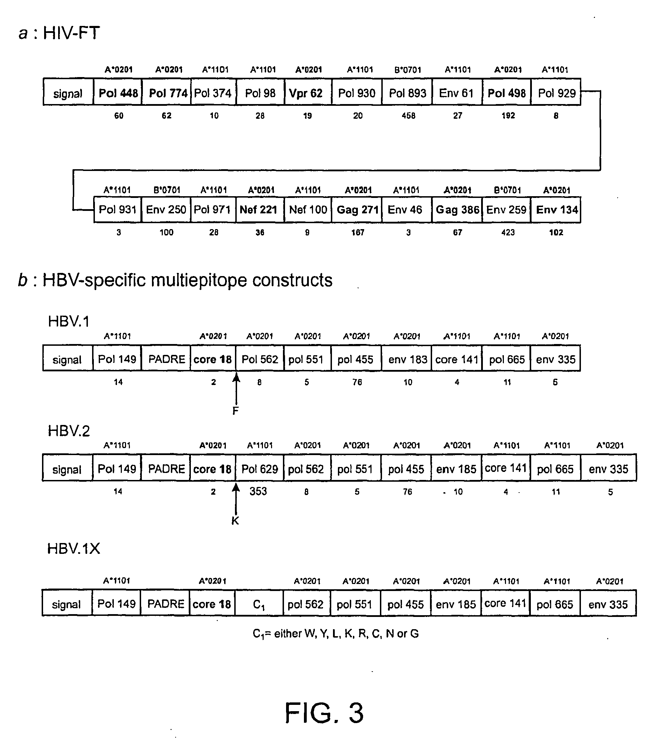

Figure 3 depicts the structure of multiepitope DNA constructs. The HLA restriction is shown above each epitope, the A*0201 epitopes are bolded. The HLA binding affinity (IC₅₀ nM) is provided below each epitope. (*a*) Schematic of HIV-FT illustrating order of the encoded epitopes. (b) Schematics of the of the HBV-specific constructs. The C+1 amino acid relative to Core 18 is indicated with an arrow. The HBV-specific constructs with single amino acid insertions at the C₁ position of Core 18 are illustrated as HBV.1X.

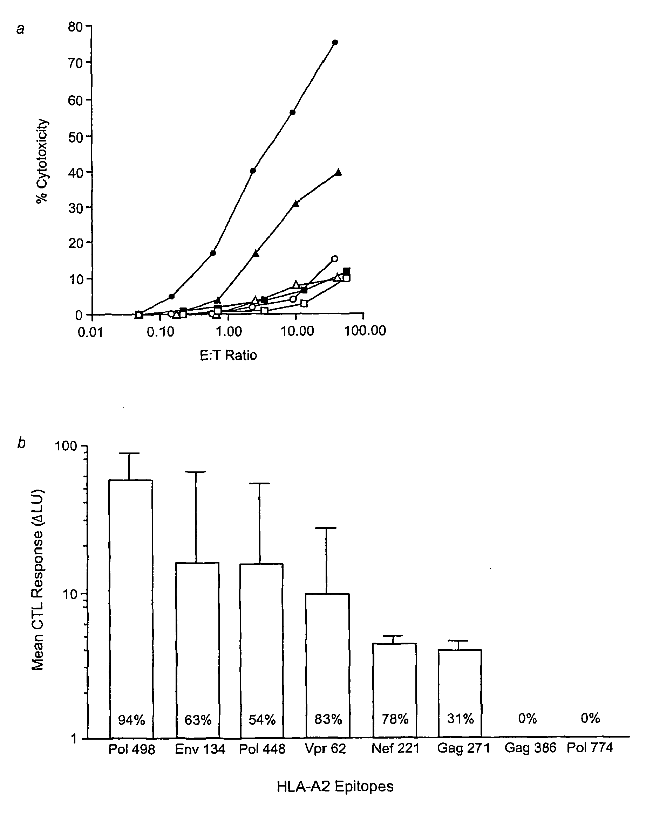

Figure 4 illustrates the immunogenicity of the HLA-A*0201 epitopes in HIV-FT in HLA-A*0201/K^{b} transgenic mice. (a) Representative CTL responses against epitopes Pol 498 (circles), Vpr 62 (triangle), Gag 386 (squares). Cytotoxicity was assayed in a⁵¹Cr release assay against Jurkat-HLA-A*0201/K^{b} target cells in the presence (filled symbols) or absence (open symbols) of each peptide. (b) Summary of CTL responses of immunogenicity of HIV-FT in HLA-A*0201/K^{b} transgenic mice. Bars indicate the geometric mean CTL response of positive cultures. The frequency of positive CTL cultures is also indicated.

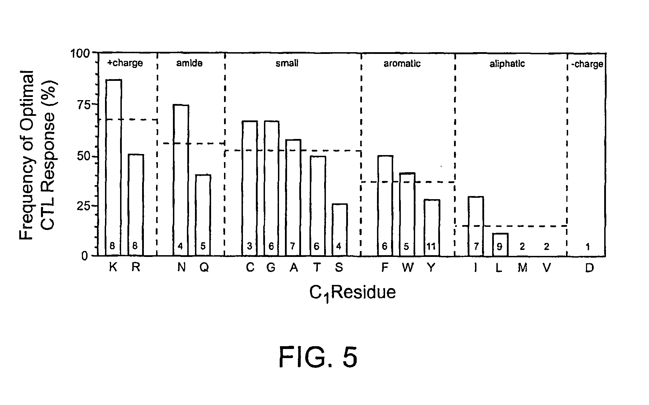

Figure 5 shows the influence of the C+1 amino acid on epitope immunogenicity. A database incorporating CTL responses from a variety of minigenes representing 94 epitope/C+1 amino acid combinations was analyzed to determine the frequency (%) of instances in which a particular combination was associated with an optimal CTL response. CTL responses were considered optimal if greater than 100 SU or 20 LU in at least 30% of the cultures measured. The number of times a given epitope/C+1 amino acid combination was observed in also provided.

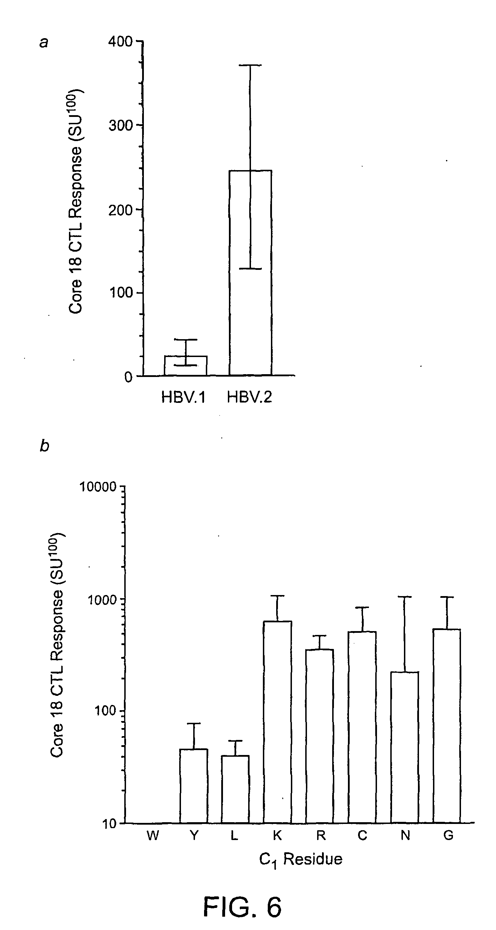

Figure 6 shows CTL responses to HBV-specific constructs (*a*) CTL responses to Core 18 epitope following DNA immunization of HLA-A*0201/K^{b} transgenic mice. (b) CTL responses to HBV Core 18 following DNA immunization of HLA-A*0201/K^{b} transgenic mice with constructs which vary by a single amino acid insertion at the C+1 position of Core 18.

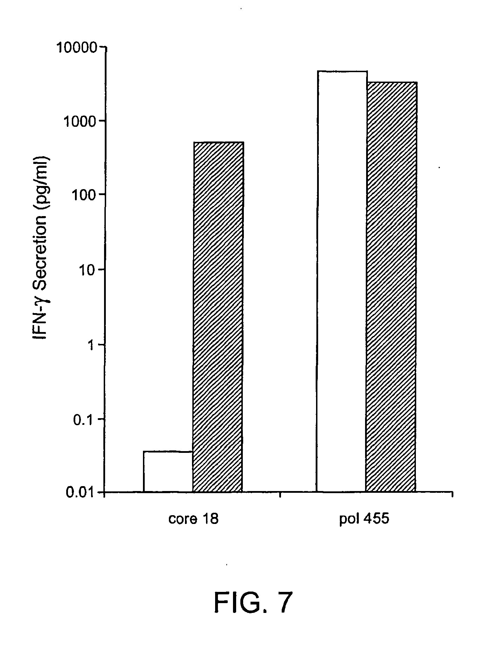

Figure 7 shows levels of HBV Core 18 presentation in HBV.1 (shaded bars) and HBV.1K (hatched bars) transfected cell lines. Epitope presentation was quantified using peptide-specific CTL lines. Presentation of HBV Pol 455 is shown for comparative purposes.

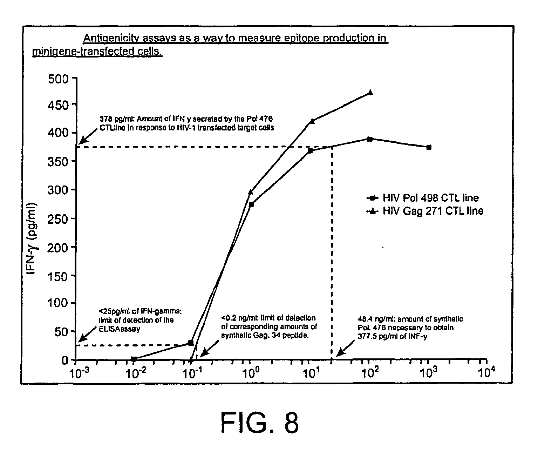

Figure 8 depicts data for 221A2K^{b} target cells transfected with the HIV-1 minigene. These transfected cells were assayed for their capacity to present epitopes to CTL lines derived from HLA transgenic mice and specific for various HIV-derived CTL epitopes. To correct for differences in antigen sensitivity of different CTL lines, peptide dose titrations, using untransfected cells as APC, were run in parallel.
Figure 9 shows HIV polyepitopic constructs optimized using the methods of the present invention.

### DESCRIPTION OF THE SPECIFIC EMBODIMENTS

### DETAILED DESCRIPTION OF THE INVENTION

### I. Introduction

The present invention relates to methods of designing multi-epitope vaccines with optimized immunogenicity. In preferred embodiments, the vaccine comprises CTL and HTL epitopes. Vaccines in accordance with the invention allow for significant, non-ethnically biased population coverage, and can preferably focus on epitopes conserved amongst different viral or other antigenic isolates. The vaccine can be optimized with regard to the magnitude and breadth of responses, and can allow for the simplest epitope configuration. Finally, general methods are provided to evaluate immunogenicityof a polyepitopic vaccine in humans.

The methods of the invention comprise designing a polyepitopic construct based on principles identified herein. In one aspect, the invention provides for simultaneous induction of responses against specific CTL and HTL epitopes, using single promoter minigene vaccines. Such minigene constructs can contain many different epitopes, preferably greater than 10, often greater than 20, 25, 30, 25, 40, 45, 50, 55, 60, 65, 70, or more.

In designing a polyepitopic construct, the following considerations are employed:
(i) the epitopes to be incorporated into the polyepitopic construct are sorted to provide an order that minimizes the number of junctional epitopes formed. In some embodiments, sorting is performed by computer. Preferably, as a secondary consideration in ordering epitopes, epitopes are positioned such that residues at the N-terminus of an epitope that promote CTL immunogenicity are juxtaposed to the C-terminus of another CTL epitope.
(ii) flanking residues that enhance immunogenicity are inserted at the flanking positions of epitopes. In particular embodiments, flanking residues are inserted at the C+1 position of CTL epitopes.
(iii) space sequences are inserted between epitopes to prevent occurance of junctional epitopes. Iin particular embodiments, the spacer sequences can also include a residue that promotes immunogenicity at the N-terminus of the linker such that the residue flanks the C-terminus of a CTL epitope.

In particular embodiments to prevent HTL junctional epitopes, a spacer composed of amino acid residues that do not correspond to any known HLA Class II anchor residue, are used, e.g, alternating G and P residues (a GP spacer) is included between two HTL epitopes.

Another aspect of the invention, (consideration (ii) above) involves the introduction or substitution of particular amino acid residues at positions that flank epitopes, e.g., a position immediately adjacent to the C-terminus of the epitope, thereby generating polyepitopic constructs with enhanced antigenicity and immunogenicity compared to constructs that do not contain the particular residue introduced or substituted at that site, *i.e.,* optimized minigenes. The methods of optimizing polyepitopic constructs comprise a step of introducing a flanking residue, preferably K, N, G, R, or A at the C+1 position of the epitope, *i.e.*, the position immediately adjacent to the C-terminus of the epitope. In an alternative embodiment, residues that contribute to decreased immunogenicity, *i.e.,* negatively charged residues, e.g., D, aliphatic residues (I, L, M, V) or aromatic non-trytophan residues, are replaced. The flanking residue can be introduced by positioning appropriate eptiopes to provide the favorable flanking residue, or be inserting a specific residue.

### CONSIDERATIONS IN DESIGNING OPTIMIZED POLYEPITOPIC VACCINE CONSTRUCTS

As noted in the background section, minigenes encoding up to 10 epitopes have been used to induce responses against a number of different epitopes. The data relating to an experimental minigene, pMin .1 has been published [Ishioka et al., J Immunol, Vol. 162(7):3915-25 (1999)]. Disclosed herein, are parameters for designing and evaluating multi-epitope constructs with optimized immunogenicity that address myriad disease indications of interest

Design parameters were identified based on a number of studies. In a preliminary evaluation of polyepitopic constructs, data on three different multi-epitope constructs, incorporating 20 to 25 different CTL epitopes each, are presented (Fig. 1). One construct is based on HIV-derived epitopes, (HIV-1), while the other two incorporate HCV-derived epitopes (HCV1 and HCV2, respectively). The immunogenicity of these different minigenes has been measured in either A2 or A11 HLA transgenic mice (A1, A24 and B7 restricted epitopes were not evaluated).

Thus, eleven days after a single i.m, DNA vaccine injection, responses against 8 to 14 different representative epitopes were evaluated following a single six day *in vitro* restimulation, utilizing assays to measure CTL activity (either chromium release or *in situ* IFN production, as described herein). Priming of epitope specific CTL could be demonstrated for 6/8 (75%), 10/14 (72%) and 13/14 (93%) of the epitopes tested in the case of HIV-1, HCV1 and HCV2, respectively. Thus, multi-epitope minigenes, capable of simultaneously priming CTL responses against a large number of epitopes, can be readily designed. However, it should be emphasized that CTL priming for some epitopes was not detected and, in several of the 36 cases considered, responses were infrequent, or varied significantly in magnitude over at least three orders of magnitude (1000-fold). These results strongly suggested that a more careful analysis and optimization of the minigene constructs was required to.

The possibility that the suboptimal performance of priming for certain epitopes might be related to minigene size was also examined. In fact, most of the published reports describe minigenes of up to ten epitopes, and the few instances in which 20-epitope minigenes have been reported, activity directed against only two or three epitopes was measured. To address this possibility, two smaller minigenes (HIV-1.1 and HIV-1.2) each encompassing ten epitopes, and corresponding to one half of the HIV-1 minigene, were synthesized and tested. Responses against four representative epitopes were measured.

| Table 1, Immunogenicity appears to be independent of minigene size. | | | | | | |
|---|---|---|---|---|---|---|
| CTL response to different minigenes | | | | | | |
| | HIV 1 | | HIV 1.1 | | HIV 1.2 | |
| CTL | (20 mer) | | (10 mer) | | (10mer) | |
| Epitope | Frequency¹⁾ | Magnitude²⁾ | Frequency | Magnitude | Frequency | Magnitude |
| Pol 774 | 0/8 | * | 0/4 | * | NA³⁾ | NA |
| Pol 498 | 18/19 | 46.7 | 4/4 | 16.4 | NA | NA |
| Gag 271 | 4/13 | 4.0 | NA | NA | 0/4 | * |
| Env 134 | 5/8 | 16.1 | NA | NA | 4/4 | 14.8 |
| 1) Represents the fraction of independent cultures yielding positive responses | | | | | | |
| 2) Lytic Units (LU) | | | | | | |
| 3) Not Applicable | | | | | | |

It was found that the responses induced by the smaller minigenes were comparable, and if anything, lower than those induced by the twenty-epitope construct (Table 1.) Accordingly, factors relating to minigene size are unlikely explanations for the observed suboptimal priming to certain epitopes and thus other parameters, disclosed herein, are used to design efficacious polyepitopic constructs.

### The minimization of junctional motifs

One of the considerations in designing polyepitopic constructs is the inadvertent creation of junctional epitopes when placing epitopes adjacent to each other. The presence of such epitopes in a minigene could significantly affect minigene performance. Strategies to guard against this undesired effect are disclosed herein for application to the development of polyepitopic or minigene vaccines. Junctional epitopes can first be minimized by sorting the epitopes to identify an order in which the numbers of junctional epitopes is minimized. Such a sorting procedure can be performed using a computer or by eye, if necessary, or depending on the number of epitopes to be included in the polyepitopic construct.

For example, a computer program that finds patterns, e.g., Panorama, can be used in the design of multi-epitope minigenes. A very large number of different epitope arrangements can be considered in designing a particular minigene construct. A computer program accepts as input, the particular set of epitopes considered, and the motifs to be scanned in order to evaluate whether there are any junctional epitopes bearing these motifs. For example, a program can simulate building a minigene, and in an euristic computational algorithm, examine epitope pairs to avoid or minimize the occurrance of junctional motifs. The program can for example, evaluate 6 X 10⁵ (about half a million) minigene configurations/second. As a less preferred alternative, as it is more time consuming, a non-computer assisted analysis can be performed.

Complete analysis of a 10-epitope construct using a computer program as described in the preceding paragraph requires examining 10 factorial ≅ 3.6X10⁶ combinations and can be completed in six seconds. A fourteen-epitope construct can be completely analyzed in a couple of days. However, analysis time goes up very rapidly as larger minigenes are considered. A complete analysis is not required and the program can be run for any desired length of time. In this case, the configuration that provides the least number of junctional epitopes is provided.

An example of the results of this type of approach is presented in Table 2. The number of junctional motifs in ten different random assortments of the same epitopes contained in the HCV1 minigene, which incorporates 25 epitopes, was the result of a two day computer analysis, is presented in this Table. In the non-optimized assortments, a large number of A2, A11 and K^{b} motifs were found, in the 25 to 38 range, with an average of 31. By comparison, only two such junctional motifs are present in the HCV 1 minigene assortment. In conclusion, a computer program can be utilized to effectively minimize the number of junctional motifs present in minigene constructs.

**Table 2. Occurrence of junctional epitopes.**

| minigene construct | selection criteria | junctional motifs |
|---|---|---|
| HCV.a | random | 33 |
| HCV.b | random | 26 |
| HCV.c | random | 28 |
| HCV.d | random | 27 |
| HCV.e | random | 30 |
| HCV.f | random | 26 |
| HCV.g | random | 38 |
| HCV.h | random | 33 |
| HCV.i | random | 33 |
| HCV.j | random | 34 |
| HCV.l | minimized | 2 |

### Eliminating Class II junctional epitopes and testing for Class II restricted responses in vivo

As a further element in eliminating junctional epitopes, spacer sequences can be inerted between two epitopes that create a junctional epitope when juxtaposed.

To correct the problem of junctional epitopes for HTL epitopes, a spacer of at least five amino acids in length is inserted between the two epitopes. The amino acids residues incorporated into such a spacer are preferably those amino acid residues that are not known to be primary anchor residues for any of the HLA Class II binding motifs. Such residues include G, P, and N. In a preferred embodiment, a spacer with the sequence GPGPG is inserted between two epitopes. Previous work has demonstrated that the GP spacer is particularly effective in disrupting Class II binding interactions [Sette et al., J. Immunol., 143:1268-73 (1989)]. All known human Class II binding motif and the mouse IA^{b} (the Class II expressed by HLA transgenic mice) do not tolerate either G or P at this main anchor positions, which are spaced four residues apart. This approach virtually guarantees that no Class II restricted epitopes can be formed as junctional epitopes.

In an example validating this design consideration, we synthesized polypeptides incorporating HIV-derived HTL epitopes. These epitopes are broadly cross-reactive HLA DR binding epitopes. It wasthen determined that these epitopes also efficiently bind the murine IA^{b} Class II molecule. A diagram illustrating the two different synthetic polypeptides considered is shown in Fig. 2a.

The first construct incorporates four different epitopes linearly arranged, while the second construct incorporates the GPGPG spacer. Synthetic peptides corresponding to the three potential junctional epitopes were also synthesized.

The capacity of 2 nanomoles of these different constructs to prime for proliferative responses to the various epitopes in IA^{b} positive mice was tested, and compared to the responses induced by equimolar amounts of a pool of the same peptides (3 micrograms of each peptide). Specifically, groups of 3 mice were injected with CFA emulsions, 11 days after injection their lymph node cells were cultured *in vitro* for an additional 3 days, and thymidine incorporation was measured in the last 24 hours of culture. It was found (Fig. 2b) that, as predicted on the basis of their high affinity IA^{b} binding capacity, all four epitopes induced good proliferation responses. Stimulation index (SI) values in the 4.9 to 17.9 range were observed when these peptides were injected in a pool. However, when the linear polypeptide incorporating the same epitopes was tested, the response directed against Pol 335 was lost. This was associated with appearance of a response directed against a junctional epitope spanning Gag 171 and Pol 335. The use of the GPGPG spacer eliminated this problem, presumably by destroying the junctional epitope, and the Pol 335 response was regained. The responses observed were of magnitude similar to those observed with the pool of isolated peptides.

These results demonstrate that responses against multiple HIV-derived Class II epitopes can be simultaneously induced, and also illustrate how IA^{b}/DR crossreactivity can be utilized to investigate the immunogenicity of various constructs incorporating HTL epitope candidates. Finally, they demonstrate that appropriate spacers can be employed to effectively disrupt Class II junctional epitopes that would otherwise interfere with effective vaccine immunogenicity.

In the case of Class I restricted responses, one case of a naturally occurring junctional epitope and the consequent inhibition of epitope specific responses has been presented by McMichael and coworkers [Tussey et al., Immunity, Vol. 3(1):65-77 (1995)]. To address the problem of junctional epitopes for Class I, similar analyses can be performed. For example, a specific computer program is employed to identify potential Class I restricted junctional epitopes, by screening for selected murine motifs and for the most common human Class I HLA A and B motifs.

Spacer sequence can also similarly be employed to prevent CTL junctional epitopes. Often, very small residues such as A or G are preferred spacer residues. G, also occurs relatively infrequently as a preferred primary anchor residue *(see, e.g.,* PCT/US00/24802) of an HLA Class I binding motif. These spacers can very in length, e.g., spacer sequences can typically be 1, 2, 3, 4, 5, 6, 7, or 8 amino acid residues in length and are sometimes longer. Smaller lengths are often preferred because of physical constraints in producing the polyepitopic construct

### The influence of flanking regions on CTL minigene immunogenicity

Another factor to be considered in desining minigenes is to insert residues that favor immunogenicity at the position flanking the C-terminus of a CTL epitope.

Disclosed herein is the identification of such residues. Flanking regions can, at least in some cases, modulate the apparent presentation of CTL epitopes. However, only limited analysis of the effect of modulation of flanking regions has heretofore been performed, and in all cases (except the study of Ishioka, 1998 [Ishioka et al., J Immunol, Vol. 162(7):3915-25 (1999)]) the epitopes were restricted by mouse MHC. This is particularly relevant as the mouse and human MHC motifs tend to differ at their C-terminus and in turn may be differentially influenced by proteosome cleavage preferences. Furthermore, few studies, if any, have considered potential differences in processing specificity between mouse and human proteosome and/or ER proteases. Thus, experiments utilizing mouse versus human epitopes could yield different results and "rules" about flanking residues. Disclosed herein are studies that identify residues that increase immunogenicity and, accordingly, residues that are inserted in polyepitopic constructs to optimize immunogenicity.

The molecular context in which an epitope was expressed often dramatically influenced the frequency and/or magnitude of priming of CTL specific for that epitope in HLA transgenic mice. Two examples are shown in Table 3.

| Table 3. Differences in effectiveness of T cell priming for specific epitopes in different minigenes. | | | | | | |
|---|---|---|---|---|---|---|
| | | Flanking | Epitope | Flanking | | Responses |
| Epitope | Minigene | N terminus | Sequence | C-terminus | Frequency | Magnitude¹⁾ |
| Core 18 | pMmin 5 | TLKAAA | FLPSDFFPSV | FLLSLG | 6/6 | 5.5 |
| | pMin1 | TLKAAA | FLPSDFFPSV | KLTPLC | 6/6 | 1074.5 |
| Core 132 | HCV1 | ILGGWV | DLMGYIPLV | YLVAYQ | 2/12 | 107.7 |
| | HCV2 | VPGSRG | DLMGYIPLV | AKFVA | 17/18 | 929.2 |

| | | | | | | |
|---|---|---|---|---|---|---|
| 1) IFN-gamma secretory units | | | | | | |

The immunogenicity of the HBV Core 18 epitope expressed in the pMin5 minigene was approximately 200-fold lower in magnitude than that observed in the case of the pMin1 minigene. Similarly, the immunogenicity of the HCV Core 132 epitope expressed in the context of the HCV 1 minigene was marginal, with significant T cell priming demonstrable in only 2 of 12 different independent CTL experiments/cultures performed. These two positive experiments yielded responses of approximately 100SU of IFN-γ. However, when the same epitope was expressed in the context of the HCV2 minigene, positive responses were observed in 17/18 cases, and with average magnitudes approximately five-fold higher.

### Immunogenicity of HIV-FT in BLA-A*0201/Kb transgenic mice

An HIV multiepitope DNA vaccine, HIV-FT (Fig. 3a) encodes 20 HIV-derived CTL epitopes. Of these 20 epitopes, eight are restricted by HLA-A*0201, nine by HLA-A* 1101 and three by HLA-B*070118. All epitopes bound their relevant restriction element with good affinity. All of the HLA-A*0201 restricted epitopes bound purified HLA-A*0201 molecules with roughly similar affinities, with IC₅₀ values in the 19-192 nM range (Fig. 3a). The HLA-A*0201 epitopes chosen for inclusion in HIV-FT are recognized in HIV-1 infected individuals and were also highly effective in priming for recall CTL responses when emulsified with IFA and utilized to prime HLA-A*0201/K^{b} transgenic mice. The construct was designed to encode the epitopes sequentially without any intervening spacer sequences between them and a consensus Igκ signal sequence was fused to the 5' end of the construct to facilitate transport of the encoded antigen into the endoplasmic reticulum (Ishioka et al., J. Immunol. 162:3915-3925, 1999).

The ability of HIV-FT to prime for recall CTL responses *in vivo* was evaluated by intramuscular immunization of HLA-A*0201/K^{b} transgenic mice. Splenocytes from animals immunized with 100µg of HIV-FT plasmid DNA were stimulated with each of the HLA-A*0201 epitopes encoded in HIV-FT and assayed for peptide-specific CTL activity after six days of culture. Representative CTL responses against three of the epitopes in HIV-FT are shown in Fig. 4a. To more conveniently compile results from different experiments the percent cytotoxicity values for each splenocyte culture were expressed in lytic units as previously described (Vitiello, et al., J. Clin. Invest 95:341-349, 1995). Of the eight HLA-A*0201 restricted epitopes encoded in HIV-FT, Pol 498, Env 134, Pol 448, Vpr 62, Nef 221, and Gag 271, primed for CTL responses following DNA immunization, (Fig. 4b). The magnitude of the CTL responses varied over greater than a 10-fold range, from as high as nearly 50 LU against Pol 498, too as little as 4 LU against Nef 221 and Gag 271. Similarly, the frequency of recall CTL responses varied between epitopes, with the Pol 498 epitope inducing responses in 94% of the cases while CTL responses to Gag 271 were detected in only 31 % of the experiments. In conclusion, DNA immunization with HIV-FT, which sequentially encodes the epitopes without any spacer amino acids, resulted in the induction of recall CTL responses against the majority of the epitopes analyzed. However, the magnitude and the frequency of the responses varied greatly between epitopes.

### Correlation between epitope immunogenicity and levels of HIV-FT epitopepresentation in transfected cell lines

Tthe differential immunogenicity of the HLA-A*0201 epitopes in HIV-FT was then assessed. Differential MHC binding affinity could be excluded as all of the epitopes bind HLA-A*0201 with high affinity (Fig. 3a). In addition, lack of a suitable repertoire of TCR specificities in HLA-A*0201/K^{b} transgenic mice could also be excluded since all epitopes yielded comparable CTL responses following immunization of HLA transgenic mice with the optimal preprocessed peptide emulsified in IFA. Variations in the relative amounts of each epitope presented for T cell recognition may at least in part account for the differences in epitope immunogenicity.

To test this, Jurkat cells, a human T cell line, expressing the HLA-A*0201/K^{b} gene (Vitiello et al., J. Exp. Med. 173, 1007-1015,1991) were transfected with the HIV-FT expressed in an episomal vector. A human cell line was selected for use to eliminate any possible artifacts that may be associated with differences in the processing capabilities between humans and mice. This transfected cell line matches the MHC presentation with the antigen processing capabilities and provides possible support for the subsequent development of CTL epitope-based DNA vaccines for use in humans.

Peptide-specific CTL lines detected presentation in the transfected targets of four of the HLA-A*0201 epitopes encoded in the HIV-FT, Pol 498, Env 134, Pol 448 and Nef 221. To quantitate the level at which each of these epitopes was produced and presented, the CTL lines specific for the various epitopes were incubated with untransfected targets and variable amounts of each epitope. These CTL dose response curves were utilized as standard curves to determine the peptide concentration inducing levels of IFN-γ secretion equivalent to those observed in response to the HIV-FT transfected target cells. This value is referred to as a "peptide equivalent dose" and taken as a relative measure of the amount of epitope presented on the transfected cell.

Table 5 summarizes the findings of this analysis for seven of the HLA-A*0201 epitopes encoded in the HIV-FT. Peptide equivalent doses varied from a high of 33.3 ng/ml for Nef 221 to less than 0.4 ng/ml peptide equivalents for epitopes Gag 271, Gag 386 and Pol 774. Cumulatively these results indicate that in human cells lines transfected with HIV-FT there is at least a 100-fold variation exists in the levels of presentation of the different HLA-A*0201 restricted epitopes. All of the epitopes that were presented at detectable levels in antigenicity assays were also immunogenic *in vivo.* The only epitope that was immunogenic and not antigenic was Gag 271. In this case immunization of HLA-A*0201/Kb transgenic mice with HIV-FT induced a weak CTL response in less than a third of the cultures tested. The other two epitopes, which were presented below the limit of sensitivity for the antigenicity analysis, Gag 386 and Pol 774, were non-immunogenic. In conclusion these results suggest that that the heterogeneity in CTL responses induced by HIV-FT immunization can at least in part be attributed to suboptimal epitope presentation.

**Table 5: Comparison of HIV-FT immunogenicity and antigenicity**

| Epitope | HIV-FT Immunogenicity | | HIV-FT Antigenicity | |
|---|---|---|---|---|
| | magnitude¹ | frequency² | Peptide Equivalents³ | n⁴ |
| Pol 498 | 58.8 (2.2) | 94% (16/17) | 23.8 (2.() | 4 |
| Env 134 | 16.1 (5.0) | 63% (5/8) | 6.2 (1.2) | 3 |
| Pol 448 | 15.7 (2.6) | 54% (7/13) | 24.7 (3.9) | 3 |
| Vpr 62 | 9.9 (1.9) | 83% (10.12) | ND | - |
| Nef 221 | 4.4 (1.3) | 78% (7/9) | 33.3 (6.0) | 3 |
| Gag 271 | 4.0 (1.4) | 31% (4/13) | <0.4 | 6 |
| Gag 386 | 0 | 0% (0/17) | <0.4 | 3 |
| Pol 774 | 0 | 0% (0/8) | <0.4 | 1 |

| | | | | |
|---|---|---|---|---|
| 1 magnitude expressed as LU (ref); the correlation coefficient relative to peptide equivalents R+0.44 2 frequency of positive cultures (number cultures >2LU/total tested); the correlation coefficient relative to peptide equivalents R+0.8. 3 magnitude expressed in ng/ml 4 number of independent experiments | | | | |

### Flanking amino acids influence CTL epitope immunogenicity in vivo following vaccination

As described herein, the particular amino acids flanking individual CTL epitopes is one factor that influences the efficiency with which an epitope is processed by altering the susceptibility the antigen to proteolytic cleavage. To examine the influence of flanking amino acids on epitope immunogenicity, immunogenicity data was obtained from HLA-A*0201, -A*1101 and-B*0701 transgenic mice immunized with a number unrelated experimental multiepitope DNA constructs encoding minimal CTL epitopes without intervening sequences. A database representing 94 different epitope/flanking residue combinations was compiled to determine the possible influence the immediately flanking amino acids on epitope immunogenicity. A given epitope and flanking amino acid combination was included only once to prevent artificial skewing of the analysis because of redundancies. Epitope immunogenicity in HLA transgenic was considered optimal if greater than 100 SU or 20 LU in at least 30% of the cultures measured. CTL responses were typically scored in one of four categories: (+++), outstanding-more than 200 LU or 1000 SU; (++), good-20-200 LU or 100-1000 SU; (+), intermediate-2 to 20 LU or 10 to 100 SU; and (+/-), weak or negative-less than 2 LLU or 10 SU. The numbers of optimal versus sub-optimal responses were categorized based on the chemical type of amino acid in the flanking positions and the significance of differences were determined using a chi-square test.

This analysis did not find any associations between the type of amino acids present at the amino-terminus of an epitope and immunogenicity. However, significant effects of the carboxyl-terminus flanking residue, the C+1 residue, were identified. Positively charged amino acids, K or R were most frequently associated with optimal CTL responses, a frequency of 68% (Fig 5). The presence of amino acids N and Q at the C+1 residue was also associated with strong CTL responses in 55.5% of the cases examined; when epitopes were flanked at the C+1 position by N, they induced optimal CTL responses in 3/4 cases. In general, small residues such as C, G, A, T, and S promoted intermediate CTL responses inducing strong responses in 54% of the combinations available for analysis. Conversely, epitopes flanked by aromatic and aliphatic amino acids induced optimal *in vivo* responses in only 36% and 17% of the cases, respectively. The negatively charged residue, D, yielded a suboptimal CTL response. The influence of C+1 amino acid on epitope immunogenicity was found to be statistically significant using a chi-square test (P<0.03). No significant influence on epitope immunogenicity was noted when similar analysis was performed for C-terminal residues more distal than the C+1 position.

### Direct evaluation of the effect of the C1 residue on epitope immunogenicity

To directly evaluate the effect of preferred versus deleterious types of amino acids in the C+1 flanking position, two multiepitope constructs, referred to as HBV.1 and HBV.2 (Fig 3b) were also evaluated. As with HIV-FT, these HBV constructs encode the epitopes sequentially without intervening spacers. Indeed, the HBV.1 and HBV.2 were generated by replacing the HIV-1 epitopes in pMin1, a experimental multiepitope construct previously characterized (Ishioka, *supra)* with similar HBV-derived epitopes.

For HBV.1, the HIV-1 epitope directly following the highly immunogenic HBV Core 18 epitope was replaced with the HBV Pol 562 epitope. This altered the C+1 residue from a K to an F. The second construct, HBV.2, was produced by the insertion of an additional epitope, HBV Pol 629, between the HBV Core 18 and Pol 562 epitopes; a change that replaced the C+1 amino acid with a K residue. When the immunogenicity of the Core 18 epitope presented in these different contexts was evaluated in HLA-A*0201/K^{b} transgenic mice, it was determined that the Core 18 epitope was virtually non-immunogenic in HBV.1 but strongly immunogenic in HBV.2 (Fig. 6a). The reduction of *in vivo* immunogenicity for this epitope was as would be predicted by our previous analysis.

To further test the effects of the C1 flanking amino acid on CTL epitope immunogenicity, a set of constructs that differ from HBV.1 by the insertion of single amino acids at the C+1 position relative to the Core 18 epitope (Fig. 3c) was evaluated. Little or no CTL response was observed against the core 18 epitope when flanked at the C+1 position by W, Y, or L (Fig 6b). In contrast, insertion of a single K residue dramatically increased the CTL response to core 18. The responses were comparable to those observed in HBV.2 in which the core 18 epitope is flanked by pol 629, an epitope with a K at the N-terminus of the epitope. Enhancement of the core 18 CTL response was also observed to insertion of R, C, N, or G. The effect of these insertions is specific, as the immunogenicity of other epitopes within these constructs did not exhibit significant changes in CTL responses (data not shown). In conclusion, these data indicate that the C+1 amino acid can dramatically influence epitope immunogenicity.

### Variations in CTL epitope immunogenicity are correlated with the amount presented

If the variation of the immunogenicity of Core 18 associated with different C+1 residues was the result of differential sensitivity to proteolytic cleavage then large differences in the levels of epitope presentation should be detectable in different constructs. To test this, Jurkat cells, expressing the same HLA-A*0201/K^{b} gene expressed in the transgenic mice20, were transfected with an episomal vector expressing either HBV.1 or HBV.1K. The Core 18 epitope was presented at >10⁵ higher levels when a K was in the C+1 position, compared to the presence of an F in the same position (Fig. 7). It is unlikely that this difference in Core 18 presentation is attributed to differences in gene expression between target cell lines since presentation of pol 455 varied by less than ten-fold. These data demonstrate the striking effect that amino acids at the C+1 position can exert on efficiency of epitope presentation in multiepitope DNA vaccines. Thus, these data show that the immunogenicity of CTL epitopes in a DNA vaccine can be optimized through design considerations that affect the level of epitope presentation. This type of optimization is applicable to epitope-based vaccines delivered using other formats, such as viral vectors as well as other expression vectors known to those of skill in the art, since the effects are exerted after the antigen is transcribed and translated.

In summary, for flanking residues, it was found that either very small residues such as A, C or G, or large residues such as Q, W, K, or R were generally associated with good or outstanding responses. The absence of a C+1 residue because of a stop codon in the minigene, or the presence of intermediate size residues such as S or T was associated with a more intermediate response pattern. Finally, in the case of a negatively charged residue, D; aliphatic (V, I, L, M) or aromatic-non tryptophan residues (Y, F), relatively poor responses were observed. These results show that the particular residue flanking the epitope's C-terminus can dramatically influence the response frequency and magnitude. Flanking residues at the C+1 position can also be introduced in combination with spacer sequences. Thus, a residue that favors immunogenicity, preferably, K, R, N, A, or G, is included as a flanking residue of a spacer.

### SORTING AND OPTIMIZATION OF POLYEPITOPIC CONSTRUCTS

To develop polyepitopic constructs using the invention, the epitopes for inclusion in the polyepitopic construct are sorted and optimized using the parameters defined herein. Sorting and optimization can be performed using a computer or, for fewer numbers of epitopes, not using a computer.

Computerized optimization can typically be performed as follows. The following provides an example of a computerized system that identifies and optimizes, *i.e.*, provides for a minimal number of junctional epitopes, and a maximal number of flanking residues, epitope combinations.

One component of sorting the epitopes is a "Junctional Analyzer". This program, for example, uses a text file to specify parameters for its operation. Five types of input data are provided to the program: 1) A set of peptides to process. 2) A set of weight for each amino acid when it appears in a C+1 and N-1 position. 3) A set of motifs to use in detecting junctions. 4) The maximum number of amino acids to insert between each pair of peptides. 5) Other values to control operation of the program. The program evaluates all possible pairs of peptides and computes the number of junctions. the C+1 and N-1 weights and combines them according to a predefined equation. Currently the equation is just the product of the two weights divided by the number of junctions. If the number of junctions is zero the product is divided by 0.5. Other equations for evaluating peptide pairs can be easily added to the program at any time. The output from this program is a text file that list for each pair of peptides the insertion that yields the maximum function result. The file also includes the original list of peptides and commands that control the operation of either of two programs that perform the next step in processing. These two programs are "Exhaustive J Search" and "Stochastic J Search".

"Exhaustive J Search" looks at all permutations of the peptides arid selects the ones that have the largest sum of the function result. This program finds the permutation with the largest function sum. However, due to the factorial nature of permutations it can only be used for a maximum of 12 or 13 peptides. The estimated running time for 13 peptides is 2.9 hours and would be approximately 40 hours for 14 peptides. "Stochastic J Search" is designed to search many areas of the permutation sequence and report the best function sum that it finds. By reporting only permutations that meet or exceed the current maximum function total, it is possible to search a much broader area of the permutation sequence. This technique has been successful with as many as 20 peptides. The time to perform an exhaustive search of 20 peptides would be on the order of 1.3 × 105 years.

The program can work as follows:
These parameters are input into the program:
   1. The epitopes to sort
   2. The amino acid C+1 & N-1 "weights", as decribed above
   3. Motifs for detecting junctional epitopes
   4. Maximum spacing residues
   5. Parameters to control program options
The Junctional Analyzer Program then performs the following:
   1. Generates all epitope pairs
   2. For each pair at peptides determine the set of insertions that results in the minimum number of junctional epitopes and the maximum effect from the C+1 and N-1 contribution of spacing residues.
   3. Outputs the optimum spacing residues for each pair and the value of the optimizing junction.

If 14 or more epitopes are to be included in the polyepitopic construct, A stochastic Search is used. A Stochastic Search Program uses a Monte Carlo technique, known to those of skill in the art, to examine many regions at the permutation space to find the best estimate the optimum arrangement of the peptides.

If less than 14 epitopes are to be included, an Exhaustive Search Program is used. The Exhaustive Search Program examines all permutations of the epitopes making up the ppolyepitopic construc to find the one with the best value for the sum of the optimizing function for all pairs of epitopes. This is guaranteed to find the "best" permutation since all are examined.

The Program Output provides a list of the best arrangements of the epitopes. Since many permutations have the same value of the evaluation function several are generated so that other factors can be considered in choosing the optimum arrangement.

Examples of polyepitopic constructs generated using this system of program analysis are provided in Figure 9.

Other factors such as charge distribution, hydrophobic/hydrophilic region analysis, or folding prediction could also be incorporated into the evaluation function to further optimize the minigene constructs.

Macromolecular structures such as polypeptide structures can be described in terms of various levels of organization. For a general discussion of this organization, *see, e.g.,* Alberts et al., Molecular Biology of the Cell (3rd ed., 1994) and Cantor and Schimmel, Biophysical Chemistry Part I: The Conformation of Biological Macromolecules (1980). "Primary structure" refers to the amino acid sequence of a particular peptide. "Secondary structure" refers to locally ordered, three dimensional structures within a polypeptide. These structures are commonly known as domains. Domains are portions of a polypeptide that form a compact unit of the polypeptide. Typical domains are made up of sections of lesser organization such as stretches of β-sheet and α-helices. "Tertiary structure" refers to the complete three dimensional structure of a polypeptide monomer. "Quaternary structure" refers to the three dimensional structure formed by the noncovalent association of independent tertiary units.

Structural predictions such as charge distribution, hydrophobic/hydrophilic region analysis, or folding predictions can be performed using sequence analysis programs known to those of skill in the art, for example, hydrophobic and hydrophilic domains can be identified (*see, e.g.,* Kyte & Doolittle, J. Mol. Biol. 157:105-132 (1982) and Stryer, Biochemistry (3rd ed. 1988); *see also* any of a number of Internet based sequence analysis programs, such as those found at dot.imgen.bcm.tmc.edu

A three-dimensional structural model of a polyepitopic construct can also be generated. This is generally performed by entering amino acid sequence to be analyzed into the computer system. The amino acid sequence represents the primary sequence or subsequence of the protein, which encodes the structural information of the protein. The three-dimensional structural model of the protein is then generated by the interaction the computer system, using software known to those of skill in the art.

The amino acid sequence represents a primary structure that encodes the information necessary to form the secondary, tertiary and quaternary structure of the protein of interest. The software looks at certain parameters encoded by the primary sequence to generate the structural model. These parameters are referred to as "energy terms," and primarily include electrostatic potentials, hydrophobic potentials, solvent accessible surfaces, and hydrogen bonding. Secondary energy terms include van der Waals potentials. Biological molecules form the structures that minimize the energy terms in a cumulative fashion. The computer program is therefore using these terms encoded by the primary structure or amino acid sequence to create the secondary structural model.

The tertiary structure of the protein encoded by the secondary structure is then formed on the basis of the energy terms of the secondary structure. The user can enter additional variables such as whether the protein is membrane bound or soluble, its location in the body, and its cellular location, e.g., cytoplasmic, surface, or nuclear. These variables along with the energy terms of the secondary structure are used to form the model of the tertiary structure. In modeling the tertiary structure, the computer program matches hydrophobic faces of secondary structure with like, and hydrophilic faces of secondary structure with like.

Those polyepitopic constructs that are most readily accessible to the HLA processing apparatus are then selected.

### II. Assessment Of Immunogenicity Of Multi-Epitopic Vaccines

The development of multi-epitope minigenes represents a unique challenge, because the species-specificity of the peptide binding to MHC. Different MHC types from different species tend to bind different sets of peptides [Rammensee et al., Immunogenetics, Vol. 41(4):178-228 (1995)]. As a result, it is not possible to test in regular laboratory animals a construct composed of human epitopes. Alternatives to overcome this limitation are generally available. They include: 1) testing analogous constructs incorporating epitopes restricted by non-human MHC; 2) reliance on control epitopes restricted by non human MHC; 3) reliance on crossreactivity between human and non-human MHC; 4) the use of HLA transgenic animals; and 5) antigenicity assays utilizing human cells *in vivo.* The following is a brief overview of the development of the technology for analyzing antigenicity and immunogenicity.

### Class I HLA Transgenics

The utility of HLA transgenic mice for the purpose of epitope identification [Sette et al., J Immunol, Vol. 153(12):5586-92 (1994); Wentworth et al.., Int Immunol, Vol. 8(5):651-9 (1996); Engelhard et al., J Immunol, Vol. 146(4):1226-32 (1991); Man et al., Int Immunol, Vol. 7(4):597-605 (1995); Shirai et al., J Immunol, Vol. 154(6):2733-42 (1995)], and vaccine development [Ishioka et al., J Immunol, Vol. 162(7):3915-25 (1999)] has been established. Most of the published reports have investigated the use of HLA A*0201/K^{b} mice but it should be noted that B*27, and B*3501 mice are also available. Furthermore, HLA A*11/K^{b} mice [Alexander et al., J Immunol, Vol. 159(10):4753-61 (1997)], and HLA B7/K^{b} and HLA A1/K^{b} mice have also been generated.

Data from 38 different potential epitopes was analyzed to determine the level of overlap between the A2.1-restricted CTL repertoire of A2.1/K^{b}-transgenic mice and A2.1+ humans [Wentworth et al., Eur J Immunol, Vol. 26(1):97-101 (1996)]. In both humans and mice, an MHC peptide binding affinity threshold of approximately 500 nM correlates with the capacity of a peptide to elicit a CTL response *in vivo.* A high level of concordance between the human data *in vivo* and mouse data *in vivo* was observed for 85% of the high-binding peptides, 58% of the intermediate binders, and 83% of the low/negative binders. Similar results were also obtained with HLA A11 and HLA B7 transgenic mice [Alexander et al., J Immunol, Vol. 159(10):4753-61 (1997)]. Thus, because of the extensive overlap that exists between T cell receptor repertoires of HLA transgenic mouse and human CTLs, transgenic mice are valuable for assessing immunogenicity of the polyepitopic constructs described herein.

The different specificities of TAP transport as it relates to HLA A11 mice does not prevent the use of HLA-A11 transgenic mice of evaluation of immunogenicity. While both murine and human TAP efficiently transport peptides with an hydrophobic end, only human TAP has been reported to efficiently transport peptides with positively charged C terminal ends, such as the ones bound by A3, A11 and other members of the A3 supertype. This concern does not apply to A2, A1 or B7 because both murine and human TAP should be equally capable of transporting peptides bound by A2, B7 or A1. Consistent with this understanding, Vitiello [Vitiello et al., J Exp Med, Vol. 173(4):1007-15 (1991)] and Rotzschke [Rotzschke O, Falk K., Curr Opin Immunol, Vol. 6(1):45-51 (1994)] suggested that processing is similar in mouse and human cells, while Cerundolo [Rotzschke O, Falk K., Curr Opin Immunol, Vol. 6(1):45-51 (1994)] suggested differences in murine versus human cells, both expressing HLA A3 molecules. However, using HLA A11 transgenics, expression of HLA molecules on T and B cells *in vivo* has been observed, suggesting that the reported unfavorable specificity of murine TAP did not prevent stabilization and transport of A11/K^{b} molecules *in vivo* [Alexander et al., J Immunol, Vol. 159(10):4753-61 (1997)]. These data are in agreement with the previous observation that peptides with a charged C termini could be eluted from murine cells transfected with A11 molecules [Maier et al., Immunogenetics; Vol. 40(4):306-8 (1994)]. Responses in HLA A11 mice to complex antigens, such as influenza, and most importantly to A11 restricted epitopes encoded by multi-epitope minigenes [Ishioka et al., J Immunol, Vol. 162(7):3915-25 (1999)] has also been detected. Thus, the TAP issue appears to be of minor concern with transgenic mice.

Another issue of potential relevance in the use of HLA transgenic mice is the possible influence of β2 microglobulin on HLA expression and binding specificity. It is well known that human β2 binds both human and mouse MHC with higher affinity and stability than mouse β2 microglobulin [Shields et al., Mol Immunol Vol. 35(14-15):919-28 (1998)]. It is also well known that more stable complexes of MHC heavy chain and β2 facilitate exogenous loading of MHC Class I [Vitiello et al., Science, Vol. 250(4986): 1423-6 (1990)]. We have examined the potential effect of this variable by generating mice that are double transgenics for HLA/K^{b} and human β2. Expression of human β2 was beneficial in the case HLA B7/K^{b} mice, and was absolutely essential to achieve good expression levels in the case of HLA A1 transgenic mice. Accordingly, HLA/K^{b} and β2 double transgenic mice are currently and routinely bred and utilized by the present inventors. Thus, HLA transgenic mice can be used to model HLA-restricted recognition of four major HLA specificities (namely A2, A11, B7 and A1) and transgenic mice for other HLA specificities can be developed as suitable models for evaluation of immunogenicity.

### Antigenicity testing for Class I epitopes

Several independent lines of experimentation indicate that the density of class I/peptide complexes on the cell surface may correlate with the level of T cell priming. Thus, measuring the levels at which an epitope is generated and presented on an APC's surface provides an avenue to indirectly evaluate the potency of minigene vaccines in human cells, *in vitro.* As a complement to the use of HLA Class I transgenic mice, this approach has the advantage of examining processing in human cells. [Ishioka et al., J Immunol, Vol. 162(7):3915-25 (1999)]

Several possible approaches to experimentally quantitate processed peptides are available. The amount of peptide on the cell surface can be quantitated by measuring the amount of peptide eluted from the APC surface [Sijts et al., J Immunol, Vol. 156(2):683-92 (1996); Demotz et al., Nature, Vol. 342(6250):682-4 (1989)]. Alternatively, the number ofpeptide-MHC complexes can be estimated by measuring the amount of lysis or lymphokine release induced by infected or transfected target cells, and then determining the concentration of peptide necessary to obtain equivalent levels of lysis or lymphokine release [Kageyama et al., J Immunol, Vol. 154(2):567-76 (1995)].

A similar approach has also been used to measure epitope presentation in minigene-transfected cell lines. Specifically, minigene constructs that are immunogenic in HLA transgenic mice are also processed into optimal epitopes by human cells transfected with the same minigene, and the magnitude of the response observed in transgenic mice correlates with the antigenicity observed with the transfected human target cells [Ishioka et al., J Immunol, Vol. 162(7):3915-25 (1999)].

Using antigenicity assays, a number of related minigenes differing in epitope order or flanking residues can be transfected into APCs and the impact of the aforementioned variables on epitope presentation can be evaluated. This can be a preferred system for testing where a relatively large number of different constructs need to be evaluated. Although it requires large numbers of epitope-specific CTLs protocols that allow for the generation of highly sensitive CTL lines [Alexander-Miller et al., Proc Natl Acad Sci U S A, Vol. 93(9):4102-7 (1996)] and also for their expansion to large numbers [Greenberg P.D., Riddell S.R, Science, Vol. 285(5427):546-51 (1999)] have been developed to address this potential problem.

It should also be kept in mind that, if the cell selected for the transfection is not reflective of the cell performing APC function *in vivo,* misleading results could be obtained. Cells of the B cell lineage, which are known "professional" APCs, are typically employed as transfection recipients. The use of transfected B cells of this type is an accepted practice in the field. Furthermore, a good correlation has already been noted between *in vitro* data utilizing transfected human B cells and *in vivo* results utilizing HLA transgenic mice, as described in more detail herein.

### Measuring HTL responses

In preferred embodiments, vaccine constructs are optimized to induce Class II restricted immune responses. One method of evaluating polyepitopic constructs including Class II epitopes, is to use HLA-DR transgenic mice. Several groups have produced and characterized HLA-DR transgenic mice [Taneja V., David C.S., Immunol Rev, Vol. 169:67-79 (1999)].

An alternative also exists which relies on crossreactivity which between certain human MHC molecules and particular MHC molecules expressed by laboratory animals. Bertoni and colleagues [Bertoni et al., J Immunol, Vol. 161(8):4447-55 (1998)] have noted that appreciable crossreactivity can be demonstrated between certain HLA Class I supertypes and certain PATR molecules expressed by chimpanzees. Crossreactivity between human and macaques at the level of Class II [Geluk et al., J Exp Med, Vol. 177(4):979-87 (1993)] and Class I molecules [Dzuris, et al., J. Immunol., July 1999] has also been noted. Finally, it can also be noted that the motif recognized by human HLA B7 supertype is essentially the same as the one recognized by the murine Class I L^{d} [Rammensee et al., Immunogenetics, Vol. 41(4):178-228 (1995)]. Of relevance to testing HLA DR restricted epitopes in mice, it has been shown by Wall, *et al* [Wall et al., J. Immunol., 152:4526-36 (1994)] that similarities exist in the motif of DR1 and IA^{b}. We routinely breed our transgenic mice to take advantage of this fortuitous similarity. Furthermore, we have also shown that most of our peptides bind to IA^{b}, so that we use these mice for the study of CTL and HTL immunogenicity.

### Measuring and Quantitating Immune Responses from Clinical Samples

A crucial element to assess vaccine performance is to evaluate its capacity to induce immune responses. Analyses of CTL and HTL responses against the immunogen, as well as against common recall antigens are commonly used and are known in the art. Assays employed included chromium release, lymphokine secretion and lymphoproliferation assays.

More sensitive techniques such as the ELISPOT assay, intracellular cytokine staining, and tetramer staining have become available in the art. It is estimated that these newer methods are 10- to 100-fold more sensitive than the common CTL and HTL assays [Murali-Krishna et al., Immunity, Vol. 8(2):177-87 (1998)], because the traditional methods measure only the subset of T cells that can proliferate *in vitro,* and may, in fact, be representative of only a fraction of the memory T cell compartment [Ogg G.S., McMichael A.J., Curr Opin Immunol, Vol. 10(4):393-6 (1998)]. Specifically in the case of HIV, these techniques have been used to measure antigen-specific CTL responses from patients that would have been undetectable with previous techniques [Ogg et al., Science, Vol. 279(5359):2103-6 (1998); Gray et al., J Immunol, Vol. 162(3):1780-8 (1999); Ogg et al., J Virol, Vol. 73(11):9153-60 (1999); Kalams et al., J Virol, Vol. 73(8):6721-8 (1999); Larsson et al., AIDS, Vol. 13(7):767-77 (1999); Come et al., J Acquir Immune Defic Syndr Hum Retrovirol, Vol. 20(5):442-7 (1999)].

With relatively few exceptions, direct activity of freshly isolated cells has been difficult to demonstrate by the means of traditional assays [Ogg G.S., McMichael A.J., Curr Opin Immunol, Vol. 10(4):393-6 (1998)]. However, the increased sensitivity of the newer techniques has allowed investigators to detect responses from cells freshly isolated from infected humans or experimental animals [Murali-Krishna et al., Immunity, Vol. 8(2): 177-87 (1998); Ogg G.S., McMichael A.J., Curr Opin Immunol, Vol. 10(4):393-6 (1998)]. The availability of these sensitive assays, that do not depend on an *in vitro* restimulation step, has greatly facilitated the study of CTL function in natural infection and cancer. In contrast, assays utilized as an endpoint to judge effectiveness of experimental vaccines are usually performed in conjunction with one or more *in vitro* restimulation steps [Ogg G.S., McMichael A.J., Curr Opin Immunol, Vol. 10(4):393-6 (1998)]. In fact, with few exceptions [Hanke et al., Vaccine, Vol. 16(4):426-35 (1998)] (Allen et al., submitted), freshly isolated Class I-restricted CD8+ T cells have been difficult to demonstrate in response to immunization with experimental vaccines designed to elicit CTL responses. The use of sensitive assays, such as ELISPOT or *in situ* IFN-γ ELISA, have been combined with a restimulation step to achieve maximum sensitivity; MHC tetramers are also used for this purpose.

MHC tetramers were first described in 1996 by Altman and colleagues. They produced soluble HLA-A2 Class I molecules which were folded with HIV-specific peptides containing a CTL epitope complexed together into tetramers tagged with fluorescent markers. These are used to label populations of T cells from HN-infected individuals that recognize the epitope [Ogg G.S., McMichael A.J., Curr Opin Immunol, Vol. 10(4):393-6 (1998)]. These cells were then quantified by flow cytometry, providing a frequency measurement for the T cells that are specific for the epitope. This technique has become very popular in HIV research as well as in other infectious diseases [Ogg G.S., McMichael A.J., Curr Opin Immunol, Vol. 10(4):393-6 (1998); Ogg et al., Science, Vol. 279(5359):2103-6 (1998); Gray et al., J Immunol, Vol. 162(3):1780-8 (1999); Ogg et al., J Virol, Vol. 73(11):9I53-60 (1999); Kalams et al., J Virol, Vol. 73(8):6721-8 (1999)]. However, HLA polymorphism can limit the general applicability of this technique, in that the tetramer technology relies on defined HLA/peptide combinations. However, it has been shown that a variety of peptides, including HIV-derived peptides, are recognized by peptide-specific CTL lines in the context of different members of the A2, A3 and B7 supertypes [Threlkeld et al., J Immunol, Vol. 159(4):1648-57 (1997); Bertoni et al., J Clin Invest, Vol. 100(3):503-13 (1997)]. Taken together these observations demonstrate that a T cell receptor (TCR) for a given MHC/peptide combination can have detectable affinity for the same peptide presented by a different MHC molecule from the same supertype.

In circumstances in which efficacy of a prophylactic vaccine is primarily correlated with the induction of a long-lasting memory response, restimulation assays can be the most appropriate and sensitive measures to monitor vaccine-induced immunological responses. Conversely, in the case of therapeutic vaccines, the main immunological correlate of activity can be the induction of effector T cell function, most aptly measured by primary assays. Thus, the use of sensitive assays allows for the most appropriate testing strategy for immunological monitoring of vaccine efficacy.

### ANTIGENICITY OF MULTI-EPITOPE MINIGENES IN TRANSFECTED HUMAN APCs

Antigenicity assaysare performed to evaluate epitope processing and presentation in human cells. An episomal vector to efficiently transfect human target cells with epitope-based minigene vaccines is used to perform such an analysis.

For example, 221A2K^{b} target cells were transfected with an HIV-1 minigene vaccine. The 221 A2K^{b} target cell expresses the A2K^{b} gene that is expressed in HLA transgenic mice, but expresses no endogenous Class I [Shimizu Y, DeMars R, J Immunol, Vol. 142(9):3320-8 (1989)]. These transfected cells were assayed for their capacity to present antigen to CTL lines derived from HLA transgenic mice and specific for various HIV-derived CTL epitopes. To correct for differences in antigen sensitivity of different CTL lines, peptide dose titrations, using untransfected cells as APC, were run in parallel. Representative data is presented in Fig. 8. In the case of HIV Pol 498-specific CTL, the transfected target cells induced the release of 378 pg/ml of IFN-γ. Inspection of the peptide dose responses reveals that, 48 ng/ml of exogenously added peptide was necessary to achieve similar levels of IFN-γ release. These results demonstrate that relatively large amounts of Pol 498 epitope are presented by the transfected cells, equivalent to 48 ng/ml of exogenously added peptide.

| Table 4. Comparison between antigenicity in transfected human cells and immunogenicity in HLA transgenic mice of the HIV-1 minigene | | | | |
|---|---|---|---|---|
| | **Antigenicity** | | **Immunogenicity** | |
| **Epitope** | **Peptide Equivalents¹⁾** | **n²⁾** | **% response³⁾** | **Magnitude⁴⁾** |
| HIV Pol 498 | 30.5 | (6) | 95% | 46.7 |
| HIV Env 134 | 6.2 | (3) | 62% | 16.1 |
| HIV Nef 221 | 2.1 | (5) | 82% | 3.8 |
| HIV Gag 271 | <0.2 | (6) | 31% | 4 |
| 1) ng/ml; 2) number of independent experiments; 3) % of CTL cultures yielding positive results; 4) Lytic Units | | | | |

By comparison, less than 25 pg/ml γ IFN was detected utilizing the CTL specific for the Gag 271 epitope. The control peptide titration with untransfected target cells revealed that this negative result could not be ascribed to poor sensitivity of the particular CTL line utilized, because as little as 0.2 pg/ml of "peptide equivalents" (PE) could be detected. Thus, it appears that the Gag 271 epitope is not efficiently processed and presented in the HIV-1 transfected target cells. Utilizing the "peptide equivalents" figure as an approximate quantitation of the efficiency of processing, it can be estimated that at least 200-fold less Gag 271 is presented by the transfected targets, compared to the Pol 498 epitope.

The results of various independent determinations for four different epitopes contained within HIV-1 are compiled in Table 4. The amount of each epitope produced from the HIV-1 transfected cells ranged from 30.5 PE for Pol 498, to a low of less than 0.2 PE for Gag 271. The two epitopes Env 134 and Nef 221 were associated with intermediate values, of 6.1 and 2.1 PE, respectively.

These results were next correlated with the *in vivo* immunogenicity values observed for each epitope after immunization with the HIV-1 construct. The Pol 498 epitope was also the most immunogenic, as would be predicted. The Env 134 and Nef 221 epitopes, for which intermediate immunogenicity was observed *in vivo,* were also processed *in vitro* with intermediate efficiency by the transfected human cells. Finally, the Gag 271, for which no detectable *in vitro* processing was observed was also associated with *in vivo* immunogenicity suboptimal in both frequency and magnitude.

These data have several important implications. First, they suggest that different epitopes contained within a given minigene may be processed and presented with differential efficiency. Secondly, they suggest that immunogenicity is proportional to the amount of processed epitope generated. Finally, these results provide an important validation of the use of transgenic mice for the purpose of optimization of multi-epitope vaccines destined for human use.

### III. Preparation of polyepitopic constructs

Epitopes for inclusion in the polyepitopic constructs typically bear HLA Class I or Class II binding motifs as described, for example, in PCT applications PCT/US00/27766, or PCT/US00/19774.

Multiple HLA class II or class I epitopes present in a polyepitopic construct can be derived from the same antigen, or from different antigens. For example, a polyepitopic construct can contain one or more HLA epitopes that can be derived from two different antigens of the same virus or from two different antigens of different viruses. Epitopes for inclusion in a polyepitopic construct can be selected by one of skill in the art, e.g., by using a computer to select epitopes that contain HLA allele-specific motifs or supermotifs. The polyepitopic constructs of the invention can also encode one or more broadly cross-reactive binding, or universal, HLA class II epitopes, e.g., PADRE^{™} (Epimmune, San Diego, CA), (described, for example, in U.S. Patent Number 5,736,142) or a PADRE family molecule.

Universal HLA class II epitopes can be advantageously combined with other HLA class I and class II epitopes to increase the number of cells that are activated in response to a given antigen and provide broader population coverage of HLA-reactive alleles. Thus, the polyepitopic constructs of the invention can include HLA epitopes specific for an antigen, universal HLA class II epitopes, or a combination of specific HLA epitopes and at least one universal HLA class II epitope.

HLA class I epitopes are generally about 8 to about 13 amino acids in length, in particular 8, 9, 10, or 11 amino acids in length. HLA class II epitopes are generally about 6 to 25 amino acids in length, in particular about 13 to 21 amino acids in length. An HLA class I or II epitope can be derived from any desired antigen of interest. The antigen of interest can be a viral antigen, surface receptor, tumor antigen, oncogene, enzyme, or any pathogen, cell or molecule for which an immune response is desired. Epitopes can be selected based on their ability to bind one or multiple HLA alleles. Epitopes that are analogs of naturally occuring sequences can also be included in the polyepitopic constructs described herein. Such analog peptides are described, for example, in PCT applications PCT/CTS97/03778, PCT/US00/19774, and co-pending U.S.S.N. 09/260,714 filed 3/1/99.

Polyepitopic constructs can be generated using methodology well known in the art. For example, polypeptides comprising the polyepitopic constructs can be synthesized and linked. Typically, however, polyepitopic constructs as minigenes using recombinant DNA technology.

### IV. Expression Vectors and Construction of a Minigene

The polyepitopic constructs of the invention are typically provided as an expression vector comprising a minigene encoding the polyepitopic construct. Construction of such expression vectors is described, for example in PCT/US99/10646. The expression vectors contain at least one promoter element that is capable of expressing a transcription unit encoding the minigene in the appropriate cells of an organism so that the antigen is expressed and targeted to the appropriate HLA molecule. For example, for administration to a human, a promoter element that functions in a human cell is incorporated into the expression vector.

The invention relies on routine techniques in the field of recombinant genetics. Basic texts disclosing the general methods of use in this invention include Sambrook et al., Molecular Cloning, A Laboratory Manual (2nd ed. 1989); Kriegler, Gene Transfer and Expression: A Laboratory Manual (1990); and Current Protocols in Molecular Biology (Ausubel et al., eds., 1994); Oligonucleotide Synthesis: A Practical Approach (Gait, ed., 1984); Kuijpers, Nucleic Acids Research 18(17):5197 (1994); Dueholm, J. Org. Chem. 59:5767-5773 (1994); Methods in Molecular Biology, volume 20 (Agrawal, ed.); and Tijssen, Laboratory Techniques in Biochemistry and Molecular Biology--Hybridization with Nucleic Acid Probes, e.g., Part I, chapter 2 "Overview of principles of hybridization and the strategy of nucleic acid probe assays" (1993)).

The nucleic acid encoding the epitopes are assembled in a minigene according to standard techniques. In general, the nucleic acid sequences encoding minigene epitopes are isolated using amplification techniques with oligonucleotide primers, or are chemically synthesized. Recombinant cloning techniques can also be used when appropriate. Oligonucleotide sequences are selected which either amplify (when using PCR to assemble the minigene) or encode (when using synthetic oligonucleotides to assemble the minigene) the desired epitopes.

Amplification techniques using primers are typically used to amplify and isolate sequences encoding the epitopes of choice from DNA or RNA (*see* U.S. Patents 4,683,195 and 4,683,202; PCR Protocols: A Guide to Methods and Applications (Innis et al., eds, 1990)). Methods such as polymerase chain reaction (PCR) and ligase chain reaction (LCR) can be used to amplify epitope nucleic acid sequences directly from mRNA, from cDNA, from genomic libraries or cDNA libraries. Restriction endonuclease sites can be incorporated into the primers. Minigenes amplified by the PCR reaction can be purified from agarose gels and cloned into an appropriate vector.

Synthetic oligonucleotides can also be used to construct minigenes. This method is performed using a series of overlapping oligonucleotides, representing both the sense and non-sense strands of the gene. These DNA fragments are then annealed, ligated and cloned. Oligonucleotides that are not commercially available can be chemically synthesized according to the solid phase phosphoramidite triester method first described by Beaucage & Caruthers, Tetrahedron Letts. 22:1859-1862 (1981), using an automated synthesizer, as described in Van Devanter et. al., Nucleic Acids Res. 12:6159-6168 (1984). Purification of oligonucleotides is by either native acrylamide gel electrophoresis or by anion-exchange HPLC as described in Pearson & Reanier, J. Chrom. 255:137-149 (1983).

The epitopes of the minigene are typically subcloned into an expression vector that contains a strong promoter to direct transcription, as well as other regulatory sequences such as enhancers and polyadenylation sites. Suitable promoters are well known in the art and described, e.g., in Sambrook *et al.* and Ausubel *et al.* Eukaryotic expression systems for mammalian cells are well known in the art and are commercially available. Such promoter elements include, for example, cytomegalovirus (CMV), Rous sarcoma virus LTR and SV40.

The expression vector typically contains a transcription unit or expression cassette that contains all the additional elements required for the expression of the minigene in host cells. A typical expression cassette thus contains a promoter operably linked to the minigene and signals required for efficient polyadenylation of the transcript. Additional elements of the cassette may include enhancers and introns with functional splice donor and acceptor sites.

In addition to a promoter sequence, the expression cassette can also contain a transcription termination region downstream of the structural gene to provide for efficient termination. The termination region may be obtained from the same gene as the promoter sequence or may be obtained from different genes.

The particular expression vector used to transport the genetic information into the cell is not particularly critical. Any of the conventional vectors used for expression in eukaryotic cells may be used. Expression vectors containing regulatory elements from eukaryotic viruses are typically used in eukaryotic expression vectors, e.g., SV40 vectors, CMV vectors, papilloma virus vectors, and vectors derived from Epstein Bar virus.

The polyepitopic constructs of the invention can be expressed from a variety of vectors including plasmid vectors as well as viral or bacterial vectors. Examples of viral expression vectors include attenuated viral hosts, such as vaccinia or fowlpox. As an example of this approach, vaccinia virus is used as a vector to express nucleotide sequences that encode the peptides of the invention. Upon introduction into a host bearing a tumor, the recombinant vaccinia virus expresses the immunogenic peptide, and thereby elicits a host CTL and/or HTL response. Vaccinia vectors and methods useful in immunization protocols are described in, e.g., U.S. Patent No. 4,722,848.

A wide variety of other vectors useful for therapeutic administration or immunization, e.g. adeno and adeno-associated virus vectors, retroviral vectors, non-viral bectors such as BCG (Bacille Calmette Guerin), Salmonella typhi vectors, detoxified anthrax toxin vectors, and the like, will be apparent to those skilled in the art.

Immunogenicity and antigenicity of the polyepitopic constructs are evaluated as described herein.

### Targeting Sequences

The expression vectors of the invention encode one or more MHC epitopes operably linked to a MHC targeting sequence. The use of a MHC targeting sequence enhances the immune response to an antigen, relative to delivery of antigen alone, by directing the peptide epitope to the site of MHC molecule assembly and transport to the cell surface, thereby providing an increased number of MHC molecule-peptide epitope complexes available for binding to and activation of T cells.

MHC class I targeting sequences can be used in the present invention, e.g., those sequences that target an MHC class I epitope peptide to a cytosolic pathway or to the endoplasmic reticulum (*see, e.g.,* Rammensee et al., Immunogenetics 41:178-228 (1995)). For example, the cytosolic pathway processes endogenous antigens that are expressed inside the cell. Although not wishing to be bound by any particular theory, cytosolic proteins are thought to be at least partially degraded by an endopeptidase activity of a proteasome and then transported to the endoplasmic reticulum by the TAP molecule (transporter associated with processing). In the endoplasmic reticulum, the antigen binds to MHC class I molecules. Endoplasmic reticulum signal sequences bypass the cytosolic processing pathway and directly target endogenous antigens to the endoplasmic reticulum, where proteolytic degradation into peptide fragments occurs. Such MHC class I targeting sequences are well known in the art, and include, e.g., signal sequences such as those from Ig kappa ,tissue plasminogen activator or insulin. A preferred signal peptide is the human Ig kappa chain sequence. Endoplasmic reticulum signal sequences can also be used to target MHC class II epitopes to the endoplasmic reticulum, the site of MHC class I molecule assembly.

MHC class II targeting sequences can also be used in the invention, e.g., those that target a peptide to the endocytic pathway. These targeting sequences typically direct extracellular antigens to enter the endocytic pathway, which results in the antigen being transferred to the lysosomal compartment where the antigen is proteolytically cleaved into antigen peptides for binding to MHC class II molecules. As with the normal processing of exogenous antigen, a sequence that directs a MHC class II epitope to the endosomes of the endocytic pathway and/or subsequently to lysosomes, where the MHC class II epitope can bind to a MHC class II molecule, is a MHC class II targeting sequence. For example, group of MHC class II targeting sequences useful in the invention are lysosomal targeting sequences, which localize polypeptides to lysosomes. Since MHC class II molecules typically bind to antigen peptides derived from proteolytic processing of endocytosed antigens in lysosomes, a lysosomal targeting sequence can function as a MHC class II targeting sequence. Lysosomal targeting sequences are well known in the art and include sequences found in the lysosomal proteins LAMP-1 and LAMP-2 as described by August *et al.* (U.S. Patent No. 5,633,234, issued May 27, 1997), which is incorporated herein by reference.

Other lysosomal proteins that contain lysosomal targeting sequences include HLA-DM. HLA-DM is an endosomal/lysosomal protein that functions in facilitating binding of antigen peptides to MHC class II molecules. Since it is located in the lysosome, HLA-DM has a lysosomal targeting sequence that can function as a MHC class II molecule targeting sequence (Copier et al., J. Immunol. 157:1017-1027 (1996), which is incorporated herein by reference).

The resident lysosomal protein HLA-DO can also function as a lysosomal targeting sequence. In contrast to the above described resident lysosomal proteins LAMP-1 and HLA-DM, which encode specific Tyr-containing motifs that target proteins to lysosomes, HLA-DO is targeted to lysosomes by association with HLA-DM (Liljedahl et al., EMBO J. 15:4817-4824 (1996)), which is incorporated herein by reference. Therefore, the sequences of HLA-DO that cause association with HLA-DM and, consequently, translocation of HLA-DO to lysosomes can be used as MHC class II targeting sequences. Similarly, the murine homolog of HLA-DO, H2-DO, can be used to derive a MHC class II targeting sequence. A MHC class II epitope can be fused to HLA-DO or H2-DO and targeted to lysosomes.

In another example, the cytoplasmic domains of B cell receptor subunits Ig-α and Ig-β mediate antigen internalization and increase the efficiency of antigen presentation (Bonnerot et al., Immunity 3:335-347 (1995)), which is incorporated herein by reference. Therefore, the cytoplasmic domains of the Ig-α and Ig-β proteins can function as MHC class II targeting sequences that target a MHC class II epitope to the endocytic pathway for processing and binding to MHC class II molecules.

Another example of a MHC class II targeting sequence that directs MHC class II epitopes to the endocytic pathway is a sequence that directs polypeptides to be secreted, where the polypeptide can enter the endosomal pathway. These MHC class II targeting sequences that direct polypeptides to be secreted mimic the normal pathway by which exogenous, extracellular antigens are processed into peptides that bind to MHC class II molecules. Any signal sequence that functions to direct a polypeptide through the endoplasmic reticulum and ultimately to be secreted can function as a MHC class II targeting sequence so long as the secreted polypeptide can enter the endosomal/lysosomal pathway and be cleaved into peptides that can bind to MHC class II molecules.

In another example, the Ii protein binds to MHC class II molecules in the endoplasmic reticulum, where it functions to prevent peptides present in the endoplasmic reticulum from binding to the MHC class II molecules. Therefore, fusion of a MHC class II epitope to the Ii protein targets the MHC class II epitope to the endoplasmic reticulum and a MHC class II molecule. For example, the CLIP sequence of the Ii protein can be removed and replaced with a MHC class II epitope sequence so that the MHC class II epitope is directed to the endoplasmic reticulum, where the epitope binds to a MHC class II molecule.

In some cases, antigens themselves can serve as MHC class II or I targeting sequences and can be fused to a universal MHC class II epitope to stimulate an immune response. Although cytoplasmic viral antigens are generally processed and presented as complexes with MHC class I molecules, long-lived cytoplasmic proteins such as the influenza matrix protein can enter the MHC class II molecule processing pathway (Guéguen & Long, Proc. Natl. Acad. Sci. USA 93:14692-14697 (1996)), which is incorporated herein by reference. Therefore, long-lived cytoplasmic proteins can function as a MHC class II targeting sequence. For example, an expression vector encoding influenza matrix protein fused to a universal MHC class II epitope can be advantageously used to target influenza antigen and the universal MHC class II epitope to the MHC class II pathway for stimulating an immune response to influenza.

Other examples of antigens functioning as MHC class II targeting sequences include polypeptides that spontaneously form particles. The polypeptides are secreted from the cell that produces them and spontaneously form particles, which are taken up into an antigen-presenting cell by endocytosis such as receptor-mediated endocytosis or are engulfed by phagocytosis. The particles are proteolytically cleaved into antigen peptides after entering the endosomal/lysosomal pathway.

One such polypeptide that spontaneously forms particles is HBV surface antigen (HBV-S) (Diminsky et al., Vaccine 15:637-647 (1997); Le Borgne et al., Virology 240:304-315 (1998)), each of which is incorporated herein by reference. Another polypeptide that spontaneously forms particles is HBV core antigen (Kuhröber et al., International Immunol. 9:1203-1212 (1997)), which is incorporated herein by reference. Still another polypeptide that spontaneously forms particles is the yeast Ty protein (Weber et al., Vaccine 13:831-834 (1995)), which is incorporated herein by reference. For example, an expression vector containing HBV-S antigen fused to a universal MHC class II epitope can be advantageously used to target HBV-S antigen and the universal MHC class II epitope to the MHC class II pathway for stimulating an immune response to HBV.

### Administration In Vivo

The invention also provides methods for stimulating an immune response by administering an expression vector of the invention to an individual. Administration of an expression vector of the invention for stimulating an immune response is advantageous because the expression vectors of the invention target MHC epitopes to MHC molecules, thus increasing the number of CTL and HTL activated by the antigens encoded by the expression vector.

Initially, the expression vectors of the invention are screened in mouse to determine the expression vectors having optimal activity in stimulating a desired immune response. Initial studies are therefore carried out, where possible, with mouse genes of the MHC targeting sequences. Methods of determining the activity of the expression vectors of the invention are well known in the art and include, for example, the uptake of ³H-thymidine to measure T cell activation and the release of ⁵¹Cr to measure CTL activity as described below in Examples II and III. Experiments similar to those described in Example IV are performed to determine the expression vectors having activity at stimulating an immune response. The expression vectors having activity are further tested in human. To circumvent potential adverse immunological responses to encoded mouse sequences, the expression vectors having activity are modified so that the MHC class II targeting sequences are derived from human genes. For example, substitution of the analogous regions of the human homologs of genes containing various MHC class II targeting sequences are substituted into the expression vectors of the invention. Expression vectors containing human MHC class II targeting sequences, such as those described in Example I below, are tested for activity at stimulating an immune response in human.

The invention also relates to pharmaceutical compositions comprising a pharmaceutically acceptable carrier and an expression vector of the invention. Pharmaceutically acceptable carriers are well known in the art and include aqueous or non-aqueous solutions, suspensions and emulsions, including physiologically buffered saline, alcohol/aqueous solutions or other solvents or vehicles such as glycols, glycerol, oils such as olive oil or injectable organic esters.

A pharmaceutically acceptable carrier can contain physiologically acceptable compounds that act, for example, to stabilize the expression vector or increase the absorption of the expression vector. Such physiologically acceptable compounds include, for example, carbohydrates, such as glucose, sucrose or dextrans, antioxidants such as ascorbic acid or glutathione, chelating agents, low molecular weight polypeptides, antimicrobial agents, inert gases or other stabilizers or excipients. Expression vectors can additionally be complexed with other components such as peptides, polypeptides and carbohydrates. Expression vectors can also be complexed to particles or beads that can be administered to an individual, for example, using a vaccine gun. One skilled in the art would know that the choice of a pharmaceutically acceptable carrier, including a physiologically acceptable compound, depends, for example, on the route of administration of the expression vector.

The invention further relates to methods of administering a pharmaceutical composition comprising an expression vector of the invention to stimulate an immune response. The expression vectors are administered by methods well known in the art as described in Donnelly et al. (Ann. Rev. Immunol. 15:617-648 (1997)); Felgner et al. (U.S. Patent No. 5,580,859, issued December 3, 1996); Felgner (U.S. Patent No. 5,703,055, issued December 30, 1997); and Carson et al. (U.S. Patent No. 5,679,647, issued October 21, 1997), each of which is incorporated herein by reference. In one embodiment, the minigene is administered as naked nucleic acid.

A pharmaceutical composition comprising an expression vector of the invention can be administered to stimulate an immune response in a subject by various routes including, for example, orally, intravaginally, rectally, or parenterally, such as intravenously, intramuscularly, subcutaneously, intraorbitally, intracapsularly, intraperitoneally, intracistemally or by passive or facilitated absorption through the skin using, for example, a skin patch or transdermal iontophoresis, respectively. Furthermore, the composition can be administered by injection, intubation or topically, the latter of which can be passive, for example, by direct application of an ointment or powder, or active, for example, using a nasal spray or inhalant. An expression vector also can be administered as a topical spray, in which case one component of the composition is an appropriate propellant. The pharmaceutical composition also can be incorporated, if desired, into liposomes, microspheres or other polymer matrices (Felgner et al., U.S. Patent No. 5,703,055; Gregoriadis, Liposome Technology, Vols. I to in (2nd ed. 1993), each of which is incorporated herein by reference). Liposomes, for example, which consist of phospholipids or other lipids, are nontoxic, physiologically acceptable and metabolizable carriers that are relatively simple to make and administer.

The expression vectors of the invention can be delivered to the interstitial spaces of tissues of an animal body (Felgner et al., U.S. Patent Nos. 5,580,859 and 5,703,055). Administration of expression vectors of the invention to muscle is a particularly effective method of administration, including intradermal and subcutaneous injections and transdermal administration. Transdermal administration, such as by iontophoresis, is also an effective method to deliver expression vectors of the invention to muscle. Epidermal administration of expression vectors of the invention can also be employed. Epidermal administration involves mechanically or chemically irritating the outermost layer of epidermis to stimulate an immune response to the irritant (Carson et al., U.S. Patent No. 5,679,647).

Other effective methods of administering an expression vector of the invention to stimulate an immune response include mucosal administration (Carson et al., U.S. Patent No. 5,679,647). For mucosal administration, the most effective method of administration includes intranasal administration of an appropriate aerosol containing the expression vector and a pharmaceutical composition. Suppositories and topical preparations are also effective for delivery of expression vectors to mucosal tissues of genital, vaginal and ocular sites. Additionally, expression vectors can be complexed to particles and administered by a vaccine gun.

The dosage to be administered is dependent on the method of administration and will generally be between about 0.1 µg up to about 200 µg. For example, the dosage can be from about 0.05 µg/kg to about 50 mg/kg, in particular about 0.005-5 mg/kg. An effective dose can be determined, for example, by measuring the immune response after administration of an expression vector. For example, the production of antibodies specific for the MHC class II epitopes or MHC class I epitopes encoded by the expression vector can be measured by methods well known in the art, including ELISA or other immunological assays. In addition, the activation of T helper cells or a CTL response can be measured by methods well known in the art including, for example, the uptake of ³H-thymidine to measure T cell activation and the release of ⁵¹Cr to measure CTL activity (*see* Examples II and III below).

The pharmaceutical compositions comprising an expression vector of the invention can be administered to mammals, particularly humans, for prophylactic or therapeutic purposes. Examples of diseases that can be treated or prevented using the expression vectors of the invention include infection with HBV, HCV, HIV and CMV as well as prostate cancer, renal carcinoma, cervical carcinoma, lymphoma, condyloma acuminatum and acquired immunodeficiency syndrome (AIDS).

In therapeutic applications, the expression vectors of the invention are administered to an individual already suffering from cancer, autoimmune disease or infected with a virus. Those in the incubation phase or acute phase of the disease can be treated with expression vectors of the invention, including those expressing all universal MHC class II epitopes, separately or in conjunction with other treatments, as appropriate.

In therapeutic and prophylactic applications, pharmaceutical compositions comprising expression vectors of the invention are administered to a patient in an amount sufficient to elicit an effective immune response to an antigen and to ameliorate the signs or symptoms of a disease. The amount of expression vector to administer that is sufficient to ameliorate the signs or symptoms of a disease is termed a therapeutically effective dose. The amount of expression vector sufficient to achieve a therapeutically effective dose will depend on the pharmaceutical composition comprising an expression vector of the invention, the manner of administration, the state and severity of the disease being treated, the weight and general state of health of the patient and the judgment of the prescribing physician.

### EXAMPLES

The following examples are offered to illustrate, but no to limit the claimed invention. It is understood that the examples and embodiments described herein are for illustrative purposes only and that various modifications or changes in light thereof are suggested to persons skilled in the art and are to be included within the spirit and purview of this application and scope of the appended claims.

Examples 1-9 provide examples of assays for evaluating the immunogenicity and antigenicity of polyepitopic constructs.

### EXAMPLE 1:

### ANTIGENICITY ASSAYS

High-affinity peptide-specific CTL lines can be generated from splenocytes of transgenic mice that have been primed with DNA, peptide/IFA, or lipopeptide. Briefly, splenocytes from transgenic mice are stimulated 0.1 µg/ml peptide and LPS blasts. Ten days after the initial stimulation, and weekly thereafter, cells are restimulated with LPS blasts pulsed for 1 hour with 0.1 µg/ml peptide. CTL lines are assayed 5 days following restimulation in an *in situ* IFN-γ ELISA as described above. Alternatively, CTL lines that are derived from, *e.g.*, patients infected with the targeted pathogen or who have the targeted disease, e.g., cancer, can be used. Specific CTL lines that are not available either from transgenic mice or from patents are generated from PBMC of normal donors, drawing on the expertise in the art.

Target cells to be used in these assays are Jurkat or .221 cells transfected with A2.1/K^{b}, A11/K^{b}, A1/K^{b}, or B7/K^{b} for CTL lines derived from transgenic mice. All these cell lines are currently available to us (Epimmune Inc., San Diego, CA). In the case of human CTL lines, .221 cells transfected with the appropriate human HLA allele are utilized. We currently have .221 cells transfected with A2 and A1, and are generating A11, A24 and B7 transfectants. In an alternative embodiment, if unforeseen problems arise in respect to target cells, LPS blasts and EBV-transformed lines are utilized for murine and human CTL lines, respectively.

To assay for antigenicity, serially diluted CTLs are incubated with 10⁵ target cells and multiple peptide concentrations ranging from 1 to 10⁻⁶ µg/ml. In addition, CTLs are also incubated with target cells transfected with an episomal vector containing a minigene of interest. Episomal vectors are known in the art.

The relative amount of peptide generated by natural processing within the minigene-transfected APCs is quantitated as follows. The amount of IFN-γ generated by the CTL lines upon recognition of the transfected target cells are recorded. The amount of synthetic peptide necessary to yield the same amount of IFN-γ are interpolated from a standard curve generated when the same CTL line is incubated in parallel with known concentrations of peptide.

### EXAMPLE 2:

### MICE, IMMUNIZATIONS AND CELL CULTURES

The derivation of the HLA-A2.1/K^{b} [Vitiello et al., J Exp Med, Vol. 173(4):1007-15 (1991)] and A11/K^{b} [Alexander et al., J Immunol, Vol. 159(10):4753-61 (1997)] transgenic mice used in this study has been described. HLA B7 K^{b} and A1/K^{b} transgenic mice are available in house (Epimmune Inc., San Diego, CA). HLA DR2, DR3 and DR4 transgenic mice are obtained from C. David (Mayo Clinic). Non-transgenic H-2^{b} mice are purchased from Charles River Laboratories or other commercial vendors. Immunizations are performed as previously described [Ishioka et al., J Immunol, Vol. 162(7):3915-25 (1999)]. All cells are grown in culture medium consisting of RPMI 1640 medium with HEPES (Gibco Life Technologies) supplemented with 10% FBS, 4 mM L-glutamine, 50 µ M 2-ME, 0.5 mM sodium pyruvate, 100 µg/ml streptomycin and 100 U/ml penicillin.

HLA transgenic mice and antigenicity assays are used for the purpose of testing and optimization CTL responses. The natural crossreactivity between HLA-DR and IA^{b} can also be exploited to test HTL responses. This evaluation provides an assessment of the antigenicity and immunogenicity of polyepitopic constructs.

### EXAMPLE 3:

### PROLIFERATION ASSAYS

To assess the ability of HTL epitopes to induce an immune response, assays such as proliferation assays are often performed. For example, mouse CD4 T lymphocytes are immunomagnetically isolated from splenic single cell suspensions using DynaBeads Mouse CD4 (L3T4) (Dynal). Briefly, 2 × 10⁷ spleen cells are incubated with 5.6 × 10⁷ magnetic beads for 40 minutes at 4° C, and then washed 3 times. Magnetic beads are detached using DetachaBead Mouse CD4 (Dynal). Isolated CD4 T lymphocytes (2 × 10⁵ cells/well) are cultured with 5 × 10⁵ irradiated (3500 rad) syngeneic spleen cells in triplicate in flat-bottom 96- well microtiter plates. Purified peptides are added to wells at a final concentration of 20, 1, 0.05 and 0µ g/ml and cells are cultured for a total of 4 days. Approximately 14 hour before harvesting, 1 µCi of ³H-thymidine (ICN) is added to each well. The wells are harvested onto Unifilter GFB plates (Packard) using the Filtermate Harvester (Packard). ³H-Thymidine incorporation is determined by liquid scintillation counting using the TopCount^{TM} microplate scintillation counter (Packard).

### EXAMPLE 4:

### ⁵¹CHROMIUM RELEASE ASSAY

This assay to measure CTL activity is well known in the art. The assay quantifies the lytic activity of the T cell population by measuring the percent ⁵¹Cr released from a ⁵¹Cr-labeled target population [Brunner et al., Immunology, Vol. 14(2):181-96 (1968)]. Data derived from the chromium release assay is usually expressed either as a CTL frequency/10⁶ cell [limiting dilution analysis, LDA; [Current Protocols in Immunology, Vol 1, John Wiley & Sons, Inc., USA 1991 Chapter 3; *Manual of Clinical Laboratory Immunology,* Fifth edition, ASM Press, 1997 Section R], or by a less cumbersome quantitative assessment of bulk CTL activity [lytic Units; LU assay]. In a LU assay, the standard E:T ratio versus percent cytotoxicity data curves generated in a ⁵¹Cr-release assay are converted into lytic units (LU) per 10⁶ effector cells, with 1 LU defined as the lytic activity required to achieve 30% lysis of target cells [Wunderlick, J., Shearer, G., and Livingston, A. In: J. Coligan, A. Kruisbeek, D. Margulies, E. Shevach, and W. Strober (Eds.), Current Protocols in Immunology, Vol 1, "Assays for T cell function: induction and measurement of cytotoxic T lymphocyte activity." John Wiley & Sons, Inc., USA, p. 3.11.18]. The LU calculation allows quantifying responses and thus readily comparing different experimental values.

### EXAMPLE 5:

### IN SITU IFN-γ ELISA

An *in situ* IFN-γ ELISA assay has been developed and optimized for both freshly isolated and peptide-restimulated splenocytes *(see, e.g.,* McKinney, D.M., Skvoretz, R, Mingsheng, Q., Ishioka, G., Sette, A. Characterization Of An *In Situ* IFN-γ ELISA Assay Which is Able to Detect Specific Peptide Responses From Freshly Isolated Splenocytes Induced by DNA Minigene Immunization, in press). This assay is based on the ELISPOT assay, but utilizes a soluble chromagen, making it readily adaptable to high-throughput analysis. In both the primary and restimulation assays, this technique is more sensitive than either a traditional supernatant ELISA or the ⁵¹Cr-release assay, in that responses are observed in the *in situ* ELISA that are not detectable in these other assays. On a per cell basis, the sensitivity of the *in situ* ELISA is approximately one IFN-γ secreting Cell/10⁴ plated cells.

96-well ELISA plates are coated with anti-IFN-γ (rat anti-mouse IFN-α mAb, Clone R4-6A2, Pharmingen) overnight at 4°C, and then blocked for 2 hours at room temperature with 10% FBS in PBS. Serially diluted primary splenocytes or CTLs are cultured for 20 hours with peptide and 10⁵ Jurkat A2.1/K^{b} cells/well at 37°C with 5% CO₂. The following day, the cells are washed out and the amount of IFN-γ that had been secreted into the wells is detected in a sandwich ELISA, using biotinylated α-IFN-γ (rat anti-mouse IFN-γ mAb, Clone XMG1.2, Pharmingen) to detect the secreted IFN-γ. HRP-coupled strepavidin (Zymed) and TMB (ImmunoPure® TMB Substrate Kit, Pierce) are used according to the manufacturer's directions for color development. The absorbance is read at 450 nm on a Labsystems Multiskan RC ELISA plate reader. *In situ* IFN-γ ELISA data is evaluated in secretory units (SU), based on the number of cells that secrete 100 pg of IFN-γ in response to a particular peptide, corrected for the background amount of IFN in the absence of peptide.

### EXAMPLE 6:

### ELISPOT ASSAY

The ELISPOT assay quantifies the frequency of T cells specific for a given peptide by measuring the capacity of individual cells to be induced to produce and release specific lymphokines, usually IFN-γ. The increased sensitivity of the ELISPOT assay has allowed investigators to detect responses from cells freshly isolated from infected humans or experimental animals [Murali-Krishna et al., Immunity, Vol. 8(2):177-87 (1998); Ogg et al., Science, Vol. 279(5359):2103-6 (1998)]. The ELISPOT assays are conducted as described above for the IFN-γ ELISA until the final steps, where ExtrAvidin-AP (Sigma, 1:500 dilution) is used in place HRP-strepavidin. Color is developed using the substrate 5-BCIP (BioRad) according to the manufacturer's directions. Spots are counted using a phase contrast microscope. Alternatively, spots are counted utilizing the Zeiss KS ELISPOT reader. In this case the BCIP/NBT substrate is used.

The ELISPOT assay is routinely utilized to quantitate immune responses. The spots can be manually counted, however, in a preferred mode, a KS ELISPOT reader from Zeiss, a microscope-based system with software specifically designed to recognize and count spots is used.

### EXAMPLE 7:

### TETRAMER STAINING

Tetramer staining is a flow cytometric technique that detects epitope-specific human CD8⁺ T-lymphocytes based on the interaction between the peptide epitope, class I antigen and the T-cell receptor specific for the epitope. This assay allows for the rapid quantitation of epitope specific human CD8⁺ T-lymphocytes in freshly isolated blood samples. MHC tetramers for various HIV peptide/HLA combinations, obtained, e.g., from the NIH repository (Tetramer Core Facility: http://www.miaid.nih.gov/reposit/ tetramer/index.html). To label epitope-specific cells, 1 ×10⁶ PBMC in a 100 µl volume are incubated in the dark for 40 minutes with 5 µg/ml of the appropriate tetramer plus monoclonal antibodies that recognize human CD3 and CD8 (available in different fluorochrome-conjugated forms from commercial sources including PharMingen, San Diego, CA or BioSource, Camarillo, CA). The cells are washed and paraformaldehyde fixed prior to analysis using a FACsan or FACSCalibur flow cytometer (Becton Dickinson Immunocytometry Systems, San Jose, CA). Sample data are analyzed using CellQuest software.

### EXAMPLE 8:

### ASSAYS FROM CLINICAL SAMPLES

Various assays to evaluate the specific CD8⁺ CTL activity in frozen PBMC samples from patients or volunteers can be used. ELISPOT, chromium release, *in situ* IFN-γ release, proliferation and tetramer assays are all useful to evaluate responses from various experimental models, *e.g.,* those of murine and/or primate origin.

Experimental methods for the murine version of these assays are described above, and these are adapted to human systems as described [Livingston et al, J Immunol, Vol. 159(3):1383-92 (1997); Heathcote et al., Hepatology, Vol. 30(2):531-6 (1999); Livingston et al., J Immunol, Vol. 162(5):3088-95 (1999)] and can be further adapted a recognized by one of ordinary skill in the art. Calculations on the amounts of frozen PBMC samples necessary to complete the assays are also described greater detail in Example 14.

### EXAMPLE 9:

### TRANSGENIC ANIMALS

Transgenic mice (HLA-A2.1/K^{b} H2^{b}; HLA-A11/K^{b}; HLA-A1/K^{b}, HLA-B7/K^{b}) are immunized intramuscularly in the anterior tibialis muscle or subcutaneously in the base of the tail with doses up to 100 µg of DNA or peptide in 10-100 µl volumes. DNA is formulated in saline, and peptides in incomplete Freund's adjuvant. 11-21 days later, the animals are sacrificed using C0₂ asphyxiation, their spleens removed and used as the source of cells for *in vitro* determination of CTL function. Typically, 3-6 mice per experimental group are used. In addition, spleens from non-immunized mice are used as a source of APC for restimulation of CTL cultures. Both males and females of 8-12 weeks of age are used.

### EXAMPLE 10:

### DEMONSTRATION OF SIMULTANEOUS INDUCTION OF RESPONSES AGAINST MULTIPLE CTL AND HTL EPITOPES

### Construction and testing of CTL epitope strings:

This example provides an example of testing mutliple CTL and HTL epitopes. For example, epitope strings encompassing 10-12 different CTL epitopes under the control of a single promoter are synthesized and incorporated in a standard plasmid, pcDNA 3.1 (Invitrogen, San Diego) or a plasmid from the NGVL (National Gene Vector Laboratory, University of Michigan). These constructs include a standard signal sequence and a the universal HTL epitope, PADRE^{™}. Each set of epitopes is chosen to allow balanced population coverage. To facilitate testing and optimization, a balanced representation of epitopes that have been shown to be immunogenic in transgenic mice, and/or antigenic in humans are included.

The specific order of these CTL epitopes is chosen to minimize Class I junctional motifs by the use of the computer program, EPISORT^{™}, as described herein. If, despite best efforts regarding order optimization, potential junctional epitopes are still present in a construct in accordance with the invention, corresponding peptides are synthesized to monitor for CTL responses against such epitopes in HLA transgenic mice. Generally, minimization of junctional motifs is successful and adequate. However, if responses against any junctional epitopes are detected, these junctional epitopes are disrupted by the use of short one to two residue spacers, such as K, AK, KA, KK, or A, compatible with expected proteolytic cleavage preferences discussed in the previous sections.

Since the ultimate use of optimized constructs is a human vaccine, optimized human codons are utilized. However, to facilitate the optimization process in HLA transgenic mice, care are applied to select, whenever possible, human codons that are also optimal for mice. Human and murine codon usage is very similar (Codon usage database at http://www.kazusa.or.jp/codonn.

Human cells transfected with the various minigene vaccine constructs can be used in antigenicity assays, conducted in parallel with *in vivo* testing in HLA transgenic mice. Any potential discrepancy between minigene vaccine efficacy, due to the differential codon usage, is addressed by the availability of these two different assay systems..

Typically, antigenicity and immunogenicity testing of plasmid constructs is conducted in parallel. *In vivo* testing in transgenic mice are performed for A2, A11, B7 and A1 HLA transgenic mice. Following a protocol well established in our laboratory, cardiotoxin pretreated mice are injected i.m. with 100 ug of each plasmid and responses evaluated eleven days later [Ishioka et al., J Immunol, Vol. 162(7):3915-25 (1999)]. Assays will include ELISPOT from freshly isolated cells, as well as interferon gamma release and cytotoxicity chromium release assays from restimulated cell cultures. All of the above mentioned techniques are well established in the art. Initial testing is focused on the 8 HLA A2, 9 HLA A11 and 6 HLA B7 restricted epitopes for which immunogenicity in HLA transgenic animals has already been well demonstrated. The simultaneous measurement of responses against these epitopes is not problematic, as large colonies of transgenic mice are already established "in house" for these HLA types. Groups of four to six mice are adequate to measure responses against six to ten different epitopes, in multiple readout assays. Testing of HLA A1-restricted, HIV-derived epitopes in HLA A 1 transgenic mice is typically employed. However, should problems be encountered, antigenicity testing using human APC can be used as an alternative strategy, or, can be used to complement the transgenic mice studies.

For the purpose of extending the correlation between immunogenicity in transgenic animals and antigenicity, as noted in the studies reported herein, antigenicity testing is utilized to evaluate responses against epitopes such as Pol 498, Env 134, Nef 221, Gag 271, for which high affinity CTL lines are already available in house. For the purpose of generating additional suitable CTL lines, direct immunization of HLA transgenic mice with peptides emulsified in adjuvant, or lipopeptides are utilized, as described herein, and routinely applied in our laboratory, to generate lines for use in antigenicity assays.

Antigenicity assays are also used, as a primary readout for epitopes for which *in vivo* optimization experiments are not feasible. These epitopes include A24 and possibly A1 restricted epitopes, as well any epitope which is non-immunogenic in HLA transgenic mice. In any such cases, we use human CTL lines, generated from HIV exposed individuals. Alternatively, we generate CTL lines for *in vitro* CTL induction, using GMCSF/IL4-induced dendritic cells and peripheral blood lymphocytes [Celis et al., Proc Natl Acad Sci USA, Vol. 91(6):2105-9 (1994)].

Episomal vectors encoding the minigenes are generated and transfected into appropriate human cell lines to generate target cells. For example, the human T cell line Jurkat can be used, but lymphoblastoid cell lines have also been successfully utilized. For experiments utilizing CTL lines of human origin, well-characterized HLA-matched, homozygous, EBV cell lines are commonly used as a source of purified-MHC and as target cells and are used as recipients of the minigene transfections. For experiments utilizing CTL lines derived from HLA transgenic mice, a collection of Class I negative, EBV-transformed, mutant cell lines .221 [Shimizu Y, DeMars R, J Immunol, Vol. 142(9):3320-8 (1989)] transfected with matching HLA/K^{b} chimeric constructs are used as the recipient of the minigene transfections. Such cells effectively present peptide antigens to CTL lines [Celis et al., Proc Natl Acad Sci USA, Vol. 91(6):2105-9 (1994)].

### Construction and testing of HTL epitope strings:

Epitope strings encompassing 3-5 different HTL epitopes under the control of a single promoter are synthesized and incorporated into a standard plasmid, pcDNA 3.1 (Invitrogen, San Diego). All constructs include an Ig-α targeting sequence. To facilitate testing and optimization, each set of epitopes for a given minigene is chosen to provide a balanced representation of epitopes which are already known to be immunogenic in IA^{b} mice. The epitope order is chosen to minimize the presence of junctional epitopes by the use of the EPISORT program. In addition, all the peptides corresponding to junctions are synthesized and tested for binding to IA^{b} and, most importantly, for binding to a panel of fourteen different DR molecules, representative of the most common DR alleles worldwide [Southwood et al., J Immunol, Vol. 160(7):3363-73 (1998)]. Thus, HTL epitopes that are not directed to an antigen of interest are not created within these plasmids. However, should junctional regions with good DR binding potential (and hence, potential DR restricted immunogenicity *in vivo)* be detected, a spacer such as GPGPG is introduced to eliminate them. In all constructs, the number of Class I junctional motifs will also be minimized, as described herein.

Experimental vaccine plasmids are tested for immunogenicity using HLA DR transgenic mice and/or mice of the H2b haplotype. Proliferation and/or cytokine production are measured (IL5, IFN-γ). In a typical protocol, cardiotoxin treated mice are injected i.m. with 100 micrograms of each plasmid and responses evaluated eleven days later [Ishioka et al., J Immunol, Vol. 162(7):3915-25 (1999)].

### Testing for interactions between CTL and HTL epitopes

The activities described above yield small, functional blocks of epitopes, which are utilized to demonstrate simultaneous responses / antigenicity against all epitopes analyzable. These blocks are the subject to further optimization, as described in the next example. Using these same minigenes, immunodominance is assessed. Specifically, all the CTL plasmids are mixed together, or all the HTL plasmids are mixed together. The results obtained with the minigene pool are then compared with the results obtained with the same minigene, injected separately.

These minigene plasmids are also used to determine the effects of HTL epitopes on responses to CTL epitopes. Specifically, HTL and CTL containing plasmids are pooled and injected in mice, and CTL and HTL responses to selected epitopes are measured as described herein. Often, it is determined whether the presence, e.g., of HTL epitopes derived from the target antigen enhances CTL responses beyond the level of response attained using a plasmid-containing a pan DR binding epitope, e.g., PADRE^{™} or a PADRE family molecule, in the CTL minigene. Typically, it is also determined whether PADRE inhibits or augments responses to target antigen-derived HTL epitopes or conversely, if HTL epitopes derived from the antigen of interest inhibit or augment responses to PADRE.

Responses to a large number of epitopes is attainable using this methodology. It is possible that the pooling of constructs may inhibit responses against some of the weaker epitopes. In this case, the pooling experiments are repeated after optimization.

### EXAMPLE 11:

### OPTIMIZATION OF CTL AND HTL MINIGENE CONSTRUCTS

This example describes the optimization the CTL and HTL constructs described in Example 10. The potential influence of flanking residues on antigenicity and immunogenicity is also assessed in optimizing minigen constructs. These studies involve the inclusion of flanking residues, a synonym for which is "spacers," which have been designed to facilitate effective processing.

Such an analysis can be performed as follows. First, the results of testing of the CTL multi-epitope constructs described in Example 10 are analyzed for trends and correlations between activity and the presence of specific residues at the 3 residues flanking the epitope's N- and C-termini. Epitopes for which suboptimal CTL priming is noted, that are suboptimal with respect to magnitude of response, are the targets for flanking region optimization. For each of the CTL minigene vaccines, encoding 10-12 different CTL epitopes, 'second generation' minigene vaccines, with optimized configuration, are produced.

The first optimization design is to introduce either an Alanine (A) or Lysine (K) residue at position C+1 for all epitopes associated with suboptimal performance. A second optimization design is to introduce in the C+1 position, the residue naturally occurring in the target antigen, e.g., HIV, that are associated with antigenicity.

For selected epitopes, additional modifications are also introduced. Specifically, the effect of introducing other residue spacers at the epitope C- and N- termini are also investigated. Depending on the results of the analysis of the minigene vaccines described in Example 10, residues investigated may further include, for example, G, Q, W, S and T. If junctional epitopes are created by these modifications, alternative epitope orders eliminating the junctional epitopes, are rationally designed, as described herein. All second generation constructs are tested for antigenicity and immunogenicity, as described herein.

As a result of these modifications, variations in activity that correspond to specific modifications of the minigenes are identified. Certain modifications are found that have general, beneficial effects. To confirm this, generation and testing of additional minigene vaccines in which all epitopes (also the ones which displayed acceptable antigenicity and immunogenicity) are subject to the same modification are conducted. In some instances, increased activity is noted for some epitopes but not others, or less desirably that certain modifications increase the activity of some, but decrease the activity of other epitopes. In such cases, additional minigene vaccines are designed and tested, to retain the beneficial modifications, while excluding those alterations that proved to be detrimental or have no effect.

These minigene vaccines are designated so that: a) a minimum of predicted junctional epitopes are present; and, b) the epitopes which were not functional in the previous minigene vaccines are now in a new more efficacious context.

For HTL minigene vaccines, the data obtained from the "first generation" minigene vaccines are inspected for trends, in terms of junctional epitopes, and epitope position within the minigene, and proximity to spacers, e.g. GPGPG spacers. If specific trends are detected, second generation minigene vaccines are designed based on these trends. Alternatively, in case of minigenes yielding suboptimal activity, the potential effectiveness of other targeting strategies, such as the ones based on Ii and LAMP are reevaluated, and compared to no targeting and simple, leader sequence targeting.

When large variations in activity of either the CTL or HTL minigene vaccines described in this section are detected, the results are consistent with influences such as conformational or "long-range" effects impacting minigene activity. These variables can be analyzed by means of current molecular and cellular biology techniques. For example, cell lines transfected with the various minigenes could be analyzed for mRNA expression levels, and stability by Northern analysis or primer extension assays [Current Protocols in Molecular Biology, Vol 3, John Wiley & Sons, Inc. USA 1999].

In all minigene vaccines, an antibody tag such as MYC/his can also be included. This tag allows for testing of protein expression levels. The inclusion of MYC/his tag [Manstein et al., Gene, Vol.. 162(1):129-34 (1995)] also allows determination of the stability of the expressed products, by pulse-chase experiments. The results of these assays can then be compared with the results of the antigenicity and immunogenicity experiments. The results are inspected for the presence of trends and general rules, and correlation between the different variables examined.

### EXAMPLE 12:

### DETERMINATION OF THE SIMPLEST PLASMID CONFIGURATION CAPABLE OF EFFECTIVE DELIVERY OF SELECTED EPITOPES

The experiments described in Examples 11 and 12 are designed to address variables concerning minigene vaccine design. Ideally, a vector that can be used in humans is used through the entire program, but one DNA vaccine plasmid for the vaccine epitope optimization studies can be used and then switched to a vector suitable for human use. Actual vector selection is dependent on several variables. For example, the availability of vectors, suitable for human use, through a reliable source, such as the National Gene Vector Laboratory (University of Michigan) is a factor.

In this example, the optimized minigenes are also ligated to form larger blocks of epitopes. All constructs are preferably designed to incorporate PADRE and leader sequence targeting in the case of CTL minigenes, and Ig-α in the case of HTL epitope minigenes, respectively. Specifically, two pairs of the 10-12 CTL epitope minigenes are ligated to generate two 20-24 CTL epitope minigenes. In a situation where ligation of epitopes yields suboptimal (decreased) activity compared to the smaller minigenes, alternative combinations and orders of ligation are investigated. The specific pair of 20-24 CTL epitope minigenes yielding optimal activity are then ligated and the resulting minigene encompassing all CTL epitopes evaluated for activity. Once again up to two alternative orientations are investigated. Because of the relatively large size of this construct, the specific effect of targeting sequences are confirmed, since it is possible that leader sequence targeting are more effective on small size minigenes, while larger size constructs may be most effectively targeted by ubiquitin signals. Specifically, one construct without any specific targeting sequences is generated and compared to a construct that is targeted for degradation by the addition of a ubiquitin molecule.

A similar strategy is used for HTL. Two pairs of the 3-5 HTL epitope minigenes are ligated to generate two 7-9 HTL epitope minigenes. Once again, in a situation where ligation of these epitopes yields suboptimal (decreased) activity, alternative combinations and order of ligation are investigated. The specific pair of 7-9 CTL epitope minigenes yielding optimal activity are ligated and the resulting minigene, encompassing all HTL epitopes, is evaluated for activity. Once again, up to two alternative orientations are investigated.

Based on these results an optimized plasmid configuration capable of effective delivery of a panel, e.g., of HIV epitopes, are selected for clinical trial evaluation. Of course, epitopes from any antigen of interest (infectious or disease-associated) can be used alone or in combination. This configuration will entail one or more HTL epitope minigene(s) and one or more CTL epitope minigene(s). A combination of one long CTL and one long HTL minigene capable of effectively delivering all epitopes, is most preferable, as it simplifies further clinical development of the vaccine. In case undesirable interactions between the two minigenes are observed when co-injected, injection of the different plasmids in the same animals, but in different injection sites, or at different points in time are examined. Alternatively, if a single CTL minigene and HTL minigene encoding all the desired epitopes is not identified, pools of minigenes are considered for further development.

### EXAMPLE 13:

### EVALUATION AND CHARACTERIZATION OF CD8+ LYMPHOCYTE RESPONSES INDUCED FOLLOWING IMMUNIZATION WITH A MULTI-EPITOPE VACCINE

CD8+ lymphocyte responses are measured mostly relying on the ELISPOT technique. The ELISPOT assay is known in the art, and is regularly used in our laboratory. An automated Zeiss ELISPOT reader is also used as set forth herein. The assays utilized to measure CD8+ responses are primarily the IFN-γ ELISPOT assay, on freshly isolated cells as well as cells restimulated *in vitro* with peptide. In addition, in selected instances chromium release assays, are utilized, also with restimulated cells and the results correlated with the ones observed in the case of the ELISPOT assays. Tetramer staining on selected peptide/MI3C combinations is also be performed. It should be noted that the large number of potential HLA/peptide combinations targeted by the vaccine might makes the use of tetrameric staining reagents as the primary clinical assay impractical, as discussed in more detail in the Background section, under the subheading "Measuring and Quantitating Immune Responses from Clinical Samples."

The clinical assay is developed and validated. The timing of this activity coincides with the period of time that follows selection of a clinical vaccine minigene, and precedes the availability of actual samples from individuals enrolled in the clinical trial. Assays for CTL evaluation can be established based on experience in the art, for example, experience in establishing assays for CTL evaluations in the Phase I and II trials of the experimental HBV vaccine, Theradigm [Livingston et al, J Immunol, Vol. 159(3):1383-92 (1997); Heathcote et al., Hepatology, Vol. 30(2):531-6 (1999); Livingston et al., J Immunol, Vol. 162(5):3088-95 (1999)]. Specifically, Ficoll-purified PBMC derived from normal subjects, as well from, e.g., HN-infected, unvaccinated volunteers can be used. As noted previously, other antigenic target(s) can be used in accordance with the invention.

The epitopes utilized are a set of A2, A3 and B7 influenza-derived supertype epitopes [Gianfrani et al., Eur. J. of Immunol., (1999)], as well as the epitopes described by Ennis and collaborators [Tamura et al., J Virol, Vol. 72(11):9404-6 (1998)]. This allows validation of the assay, as well as define baseline response levels in the patient population that is treated.

After establishing the assay, clinical sample are evaluated. The general assay strategy is as follows. PBMC aliquots shipped as frozen material are used. For APC autologous PBMC pulsed with peptides are typically used, since the the epitopes employed in the polyepitopic constructs are restricted by a large collection of diverse MHC Class I alleles, making the use of typed EBV lines as APC and targets impractical.

Both unstimulated and peptide stimulated cultures are evaluated. Some responses can be detected in unstimulated cultures, but weaker responses/epitopes may require one or two *in vitro* restimulations. An assay procedure can be performed as follows. For example, if responses to approximately fifty Class I restricted epitopes are assayed, seven pools of approximately seven peptides can be used. Pools of peptides are also used for restimulation. Responses against the PADRE epitope are also measured. In the case of peptide pools yielding positive results, individual peptides are assayed to identify the epitopes responsible for the observed response (s). Positive controls include whole Tetanus Toxoid, and/or the mitogen PHA, as well as the pool of influenza virus-derived epitopes. Potential junctional epitopes are also synthesized and tested as a single pool.

Next, in the case of few selected responder peptides and individuals tetramer staining is perfomed on either fresh or restimulated samples and correlated with ELISPOT data. It should be noted that only tetramers for selected peptides bound to common alleles, such A*0201, A*0301, A*1101 and B*0702 are produced. The capacity of these tetramers to stain cells which are positive for response against the same peptides, but are restricted by other members of a given supertype, are tested. These results are interest in determining the widespread applicability of these reagents as a diagnostic/surrogate marker of therapy.

In terms of amounts of cells required for these analyses, a standard assay can include, for example seven peptides groups, one negative and two positive controls (total of 10 groups). For testing in duplicate, 2 × 10⁵ cells/well each, this corresponds to 10 × 2 × 2 × 10⁵ = 4 × 10⁶ cells. A conservative assumption of 50% recovery after thawing provides an estimate of about 10⁶ PBMC/ml of blood. Large variations in PBMC counts in clinical subjects are not expected, as all individuals are either healthy volunteers, or, in the example of HIV-infected patients, HAART therapy recipients. In conclusion, one aliquot of PBMC derived from 4-8 ml of blood should is usually sufficient for one round of assays. The assays will usually need to be repeated, either to allow for determining which peptides are positives, and/or to allow for restimulation *in vitro* for maximum sensitivity, or both. Accordingly, a total of 3-4 aliquots might be required corresponding to a total of about 20 ml of blood.

All publications, patents, and patent applications cited herein are hereby incorporated by reference in their entirety for all purposes.

### Clauses

1. A method for designing a polyepitopic construct that comprises multiple HLA epitopes and is presented to an HLA Class I processing pathway, the method comprising steps of :
   (i) sorting the multiple HLA epitopes to minimize the number of junctional epitopes;
   (ii) introducing a flanking amino acid residue selected from the group consisting of K, R, N, Q, G, A, S, C, and T at a C+1 position of an HLA epitope comprised by the polyepitopic construct;
   (iii) introducing one or more amino acid spacer residues between two epitopes comprised by the polyepitopic construct, wherein the spacer prevents the occurrence of a CTL or HTL junctional epitope; and,
   (iv) selecting one or more polyepitopic constructs that have a minimal number of junctional epitopes, a minimal number of amino acid spacer residues, and a maximum number of K, R, N, G, A, S, C, or T at a, C+1 position relative to each HLA epitope.
2. The method of clause 1, wherein the spacer residues are independently selected from residues that are not known HLA Class II primary anchor residues.
3. The method of clause 1, wherein introducing the spacer residues prevents the occurrence of an HTL epitope and further, wherein a spacer comprises at least 5 amino acid residues independently selected from the group consisting of G, P, and N.
4. The method of clause 3, wherein the spacer is GPGPG.
5. The method of clause 1, wherein introducing the spacer residues prevents the occurrence of an HTL epitope and further, wherein the spacer is 1,2,3,4,5,6,7, or 8 amino acid residues independently selected from the group consisting of A and G.
6. The method of clause 1, wherein the flanking residue is introduced at the C+1 position of a CTL epitope.
7. The method of clause 1, wherein the flanking residue is selected from the group consisting of K, R, N, G, and A.
8. The method of clause 1, wherein the flanking residue is adjacent to the spacer amino acid residues.
9. The method of clause 1, further comprising substituting an N-terminal residue of an HLA epitope that is adjacent to a C-terminus of an HLA epitope comprised by the polyepitopic construct with a residue selected from the group consisting of K, R, N, G, and A.
10. The method of clause 1, further comprising a step of predicting a structure of the polyepitopic construct, and further, wherein the selecting step further comprises selecting one or more polyepitopic constructs that, when introduced into a cell, is processed by an HLA processing pathway such that all of the eptiopes included in the polyepitopic construct are produced by the HLA processing pathway.
11. A polyepitopic construct prepared using a method in accordance with any of clauses 1-9.
12. The polyepitopic construct of clause 11, wherein the epitopes comprised by the polyepitopic construct are encoded by a minigene.
13. The polyeptiopic construct of clause 12, wherein the polyepitopic construct encoded by the minigene is HIV-TT set out in Figure 9.
14. The polyepitopic construct of clause 12, wherein the polyepitopic construct encoded by the minigene is the HIV-DG set out in Figure 9.
15. The polyepitopic construct of clause 12, wherein the polyepitopic construct encoded by the minigene is the HIV-TC construct set out in Figure 9.

## Claims

1. A multi-epitope polypeptide comprising 10 or more CTL and/or HTL epitopes; further comprising (i) at least one flanking or spacer amino acid residue at the C-terminus of one or more of the CTL epitopes, wherein said flanking or spacer amino acid residue is selected from the group consisting of K, N, G, R, S, Q, C, T and A; and/or (ii) at least one flanking or spacer amino acid residue at the C-terminus position of one or more of the HTL epitopes, wherein said flanking or spacer amino acid residue is selected from the group consisting of G, P and N; wherein said flanking or spacer amino acid residue prevents the occurrence of a CTL and/or HTL junctional epitope.

2. The multi-epitope polypeptide of claim 1, wherein the flanking or spacer amino acid residues at the C-terminus position of one or more HTL epitopes comprise at least 5 amino acid residues independently selected from the group consisting of G, P and N.

3. The multi-epitope polypeptide of claim 2, wherein the flanking or spacer amino acid residues at the C-terminus position of one or more HTL epitopes are GPGPG.

4. The multi-epitope polypeptide of claim 1, wherein the flanking or spacer amino acid residues at the C-terminus position of one or more CTL epitopes comprise 1, 2, 3, 4, 5, 6, 7, or 8 amino acid residues independently selected from the group consisting of A and G.

5. The multi-epitope polypeptide of claim 1, wherein the flanking or spacer amino acid residues at the C-terminus position of one or more CTL epitopes are selected from the group consisting of K, R, N, G and A.

6. The multi-epitope polypeptide of any of claims 1 to 5, wherein the polypeptide has been designed by a method wherein the epitopes are sorted in order to minimize the number of junctional epitopes.

7. The multi-epitope polypeptide of any of claims 1 to 6 comprising 20 or more CTL and/or HTL epitopes.

8. The multi-epitope polypeptide of claim 7 comprising 30 or more CTL and/or HTL epitopes.

9. The multi-epitope polypeptide of claim 8 comprising 40 or more CTL and/or HTL epitopes.

10. A polynucleotide minigene encoding the multi-epitope polypeptide of any of claims 1 to 9.

11. An expression vector comprising the polynucleotide minigene of claim 10.

12. A multi-epitope polypeptide encoded by the minigene HCV.1, HCV.2, HBV.1, HBV.2, or HBV.1X, as set out in Figures 1 and 3.
